(19) Europäisches Patentamt
European Patent Office
Office européen des brevets

(11) **EP 3 674 421 A1**

(12) **EUROPEAN PATENT APPLICATION**

(43) Date of publication:
**01.07.2020 Bulletin 2020/27**

(51) Int Cl.:
***C12Q 1/6886*** *(2018.01)*

(21) Application number: **18383000.9**

(22) Date of filing: **28.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Asociación Centro de Investigación Cooperativa en Biociencias - CIC bioGUNE 48160 Derio (ES)**

(72) Inventors:
• **CARRACEDO PÉREZ, Arkaitz**
  **E-48160 Derio, Vizcaya (ES)**
• **TORRANO MOYA, Verónica**
  **E-48160 Derio, Vizcaya (ES)**
• **CORTÁZAR ORTIZ, Ana Rosa**
  **E-48160 Derio, Vizcaya (ES)**
• **ASTOBIZA PÉREZ, Ianire**
  **E-48160 Derio, Vizcaya (ES)**
• **VIERA BARDÓN, Cristina**
  **E-48160 Derio, Vizcaya (ES)**

(74) Representative: **ABG Intellectual Property Law, S.L.**
**Avenida de Burgos, 16D**
**Edificio Euromor**
**28036 Madrid (ES)**

(54) **METHODS FOR THE PROGNOSIS OF PROSTATE CANCER**

(57)    The present invention relates to methods useful for predicting the outcome of a patient suffering from prostate cancer or for predicting the response to therapy of a patient suffering from prostate cancer, which comprise determining, in a sample from the patient, the ratio between the average expression levels of a first gene signature and the average expression levels of a second gene signature, wherein the first gene signature is selected from the group consisting of HI1 (i.e., the CPOX, GAL3St4, ORAI2 and NIPA1 genes) and HI2 (i.e., the KHK, NOX4, PTGES3 and RRM2 genes); wherein the second gene signature is selected from the group consisting of LO1 (i.e., the ATP8B1, CDO1, CHRNA2, GLB1L3, GNE, KATNAL2, PTGIS, SLC6A14, TP53INP2, and TRIM47 genes), LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and LO3 (i.e., the ASPA, CDO1, GFPT2, GNE, ITPKC, PAH, SIRT1 and TPMT genes). These methods can be used to identify those patients who harbour an indolent/ low risk prostate cancer, or those that are at a high risk of recurrence or metastasis. The identification of these subgroups of patients may guide the selection of therapies, improving financial and health outcomes. Moreover, the expression levels of these genes can also be used for the selection of a patient to be treated for low risk, indolent, localised, advanced, recurrent or metastatic prostate cancer. Further, the invention also relates to kits and reagents to determine the expression levels of these genes.

EP 3 674 421 A1

**Description**

**FIELD OF THE INVENTION**

The invention relates to the field of prostate cancer prognosis and, more in particular, to methods for predicting the outcome of a prostate cancer patient based on the ratio between the average expression levels of a first gene signature and the average expression levels of a second gene signature, as well as to methods for selecting a patient to be treated for low risk, indolent, localised, advanced, recurrent or metastatic prostate cancer.

**BACKGROUND ART**

Prostate cancer as an increasing socio-economic problem: Prostate cancer (PCa) is the fifth cause of death by cancer worldwide (data retrieved from the WHO), second in the male population. In the European Union, PCa exhibits the highest incidence among cancer types in men, and represents a major cause of death by cancer in the gender (www.globocan.iarc.fr).

An emerging social and clinical problem in PCa is over-diagnosis. Prostate Specific Antigen (PSA)-based screening has dramatically increased the number of diagnosed PCa cases. However, the impact of PSA screening on mortality and morbidity is far below the expectations for a diagnostic biomarker. This evidence is thought to be related to the fact that PSA complemented with imaging and pathology approaches results in the diagnosis of a fraction of PCa that would not pose a risk for the health of the patient, hereby termed indolent PCa. In the recent years, various multicentric initiatives have attempted to implement guidelines that spare patients from localized treatment in PCa, rather focusing on closing monitoring the disease (active surveillance, AS). Ascription of patients and clinicians to AS protocols is low, probably due to the low accuracy of current biomarkers in the clinic, mostly limited to the pathological features of the tumour. Therefore, molecular biomarkers with high capacity of identifying patients with indolent disease would be of high social and clinical relevance.

Opposite to the clinical problem associated to indolent PCa is the aggressive disease. A fraction of patients subjected to localized treatment (surgery or radiotherapy) eventually exhibits a rise of blood PSA, indicative of disease recurrence (termed biochemical recurrence, BCR). This evidence is sufficient to undertake alternative therapeutic measures, although a tumour mass might not be detected. Paradoxically, patients that exhibit BCR will be prone to fail to subsequent therapeutic approaches (androgen deprivation, Androgen Receptor inhibitors, chemotherapy in the form of taxols), and hence account for the majority of mortality and morbidity after they debut with metastatic disease.

Molecular subtyping for precision medicine is best explained in breast cancer, where gene expression provides key complementary information to histopathological analyses in order to reach clinical decisions for the election of the best therapeutic regime. Fifteen years ago, the first breast cancer signatures were reported. The work of Perou and Colleagues (Perou et al., Nature 2000) provided the first molecular sub-classification of breast cancer, which stands valid today. They described four major molecular subtypes, which could be distinguished based on their signatures. This type of studies prompted subsequent analyses, which resulted in the identification of gene signatures of bad prognosis, early recurrence or recurrence after therapy (van't Veer et al., Nature 2002). These and other gene signatures have led to molecular biomarkers (Mammaprint, PAM50, Oncotype DX, Kittaneh et al., Biomark Cancer 2013). These commercial systems are now routinely used in breast cancer patients in which histopathological analysis is insufficient to allow therapeutic clinical decisions. It is worth noting that, while all breast cancer patients could potentially benefit from such analyses, their limited application is due to their high cost. The field of prognostic biomarkers in prostate cancer is far less evolved than that of the breast. Importantly, unlike in breast cancer, prostate cancer clinical decisions are not yet based on these gene signatures, which strongly suggests that the commercial products available have not yet demonstrated capacity to solve this question. The main commercial options are summarised herein below:

a. **Prolaris.** This biomarker is based on the expression analysis of proliferation-based genes. Proliferation is a characteristic of fast-growing tumours. However, research in the last decade has demonstrated that more aggressive tumours undergo phases of slow proliferation and dedifferentiation, which is associated to higher resistance to external insults, such as therapeutic challenge or adaptation to hostile environments (including the establishment of metastasis). In turn, a selective focus on cell proliferation could mask tumours with the aforementioned features. The cost of Prolaris test is over 2.000$.
prolaris.com/.

b. **Oncotype DX.** This test is based on a selected set of biological features associated to prostate cancer aggressiveness, including representing a stromal response, androgen signalling, cellular organization and proliferation. While these features could associate more closely to aggressive prostate cancer, it is not projected to inform about the development of metastatic disease. The cost exceeds 1.500$.

2

www.genomichealth.com/en-US/oncotype_iq_products/oncotype_dx/oncotype_dx_prostate_cancer

c. **Decipher.** This biomarker requires tissue microdissection to select areas with the highest Gleason score (which is not routinely used in the majority of hospitals). The panel is designed to evaluate the expression of various genetic markers associated with specific of biological processes, including cell proliferation/differentiation, cell structure, adhesion and motility, the immune response, cell cycle progression and other unknown segments. As with Oncotype DX,it covers a wider range of biological aspects of the disease. The cost of this analysis also exceeds 1.500$. deciphertest.com/

d. **ProMArk.** Predicts likelihood of adverse Pathology using protein staining. Predict cancer aggressiveness in patients with biopsy Gleason Scores of 3+3 and 3+4. metamarkgenetics.com/healthcare-professionals/our-lab-services/8-protein-signature

e. **ConfirmMDx®.** Predicts likelihood of negative repeat biopsy. Our values of negative predicted value are better than the ones offered in this test. mdxhealth.com/confirmmdx-prostate-cancer

f. **4KScore.** Provides probability of aggressive cancer on initial biopsy. Relies on the measurement of four prostate-specific kallikreins in the blood: Total PSA, Free PSA, Intact PSA, and Human Kallikrein 2 (hK2). The 4Kscore test provides a man's percentage risk (reported on a scale of less than 1% to greater than 95%) of having a Gleason score 7 or higher prostate cancer diagnosed if he were to have a prostate biopsy performed. The 4Kscore test is intended to be used as a second decision point after an abnormal PSA and/or DRE, prior to performing an initial prostate biopsy or after a negative prostate biopsy. The 4Kscore Test should not be used in isolation to make a decision on whether or not to perform a prostate biopsy. The expert clinical judgment of a physician is needed to select the appropriate 4Kscore risk level and integrate other information (health status, medical history, family history of prostate cancer, PSA history, etc.) into the shared decision with the patient about having a prostate biopsy. 4kscore.com/4kscore-test-for-physicians/how-does-the-4kscore-test-work/

g. **PROGENSA PCA3 de Gen-Probe.** The test quantifies PCA3 versus PSA RNA, as PCA3 is a highly over-abundant RNA in prostate cancers, with cancer cells expressing 60- $100\times$ greater levels than normal cells. It requires a post-DRE urine sample. The final result is reported as a "PCA3 score" and is calculated as a ratio of (PCA3 RNA/ PSA RNA) x1,000. PCA3 has a pooled sensitivity of 65% and specificity of 73%, based on a meta-analysis of 46 clinical trials. www.hologic.com/package-inserts/diagnostic-products/progensa-pca3-assay

h. **The Prostate Health Index (phi)** is commercialized by Beckman Coulter Inc. and is an U.S. Food & Drug Administration (FDA) approved blood test used as an aid in distinguishing prostate cancer from benign prostatic conditions [www.beckmancoulter.com]. It is a calculation based on an algorithm combining total PSA, free PSA, and another form of PSA, namely [-2]proPSA. The test measures these analytes in blood and can be performed on standard clinical analyzers available through Beckman Coulter Inc. Thus, it provides an opportunity for most clinical labs to offer this test as a panel. However, one disadvantage is that the test kit is only offered as a bundle, and all three tests must be performed on Beckman instrumentation. Total PSA, if quantified on other analyzers, cannot be combined with the remaining analytes, so clinical labs would need to offer this test panel in addition to any other PSA testing they've already performed. The phi test outperforms PSA alone and has 90% clinical sensitivity and 31% specificity at a phi value of 27.0, allowing for a reduction of nearly 1/3 of all biopsies while detecting 90% of cancers. www.beckmancoulter.com/es/learning-and-events/webinars/immunoassay-webinars/prostate-health-index-phi-what-when-where

The available prognostic biomarkers share some common features: They rely on expression changes in genes related to well-established cancer features, they have a high cost, and they lack any coverage of metabolic genes. Accordingly, there is still a need for more efficient biomarkers with prognostic and predictive potential in order to define therapies for those patients who are at risk of developing prostate cancer.

## SUMMARY OF THE INVENTION

The present invention relates to the surprising finding that gene signatures based on metabolic gene expression are useful in the prognosis of prostate cancer patients. Importantly, the prognostic method described herein relies on the integration of gene signatures with opposing directionality in gene regulation, thus making dispensable the rate-limiting step of defining normalizers of the assay. This method can be used to determine those patients who harbour an indolent/low risk prostate cancer, or those that are at a high risk of recurrence or metastasis.

Thus, in a first aspect, the invention relates to a method for predicting the outcome of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of an increased risk of a negative outcome for the patient, or
(ii) a decreased ratio between the average expression levels of the first gene signature and the average expression levels of the second gene signature with respect to a reference value is indicative of a decreased risk of a negative outcome for the patient.

In a second aspect, the invention also relates to a method for predicting response to therapy of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of a negative response of the patient to the therapy, or
(ii) a decreased ratio between the average expression levels of the first gene signature and the average expression levels of the second gene signature with respect to a reference value is indicative of a positive response of the patient to the therapy.

In a third aspect, the invention relates to a method for selecting a patient to be treated for low risk, indolent, localised, advanced, recurrent or metastatic prostate cancer which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein an increased ratio between the average expression levels of the first gene signature and the average expression levels of the second gene signature with respect to a reference value is indicative that the patient is selected for treatment for prostate cancer.

In a fourth aspect, the invention relates to a method of treatment of low risk, indolent, localised, advanced, recurrent or metastatic prostate cancer to a patient in need thereof which comprises the administration of a therapy to said patient, wherein the patient is selected by a method which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein an increased ratio between the average expression levels of the first gene signature and the average expression levels of the second gene signature with respect to a reference value is indicative that the patient is selected for treatment for prostate cancer.

In a fifth aspect, the invention relates to kit or assay device comprising reagents adequate for the determination of the average expression level of a gene signature selected from the group consisting of HI1, HI2, LO1, LO2 and LO3, wherein the reagents are selected from the group of a set probes which specifically hybridize to the mRNA of said genes and a set of primer pairs which are capable of specifically amplifying the mRNAs of said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

Finally, in a sixth aspect, the invention relates to a kit or assay device comprising reagents adequate for the determination of the average expression level of the polypeptides encoded by the genes in a gene signature selected from the group consisting of HI1, HI2, LO1, LO2 and LO3, wherein the reagents are a set of antibodies which specifically bind to the polypeptides encoded by said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

**BRIEF DESCRIPTION OF THE FIGURES**

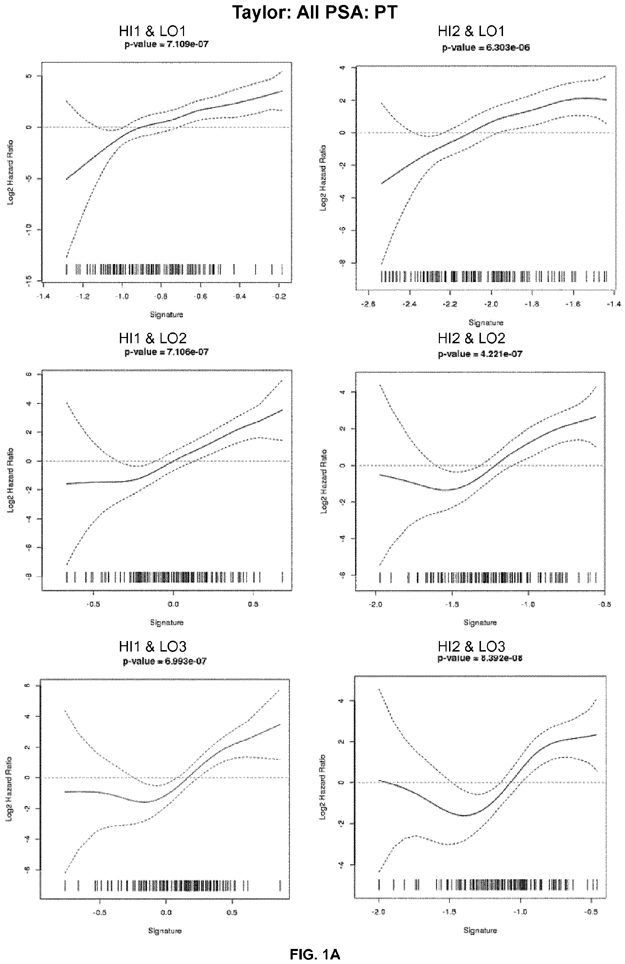

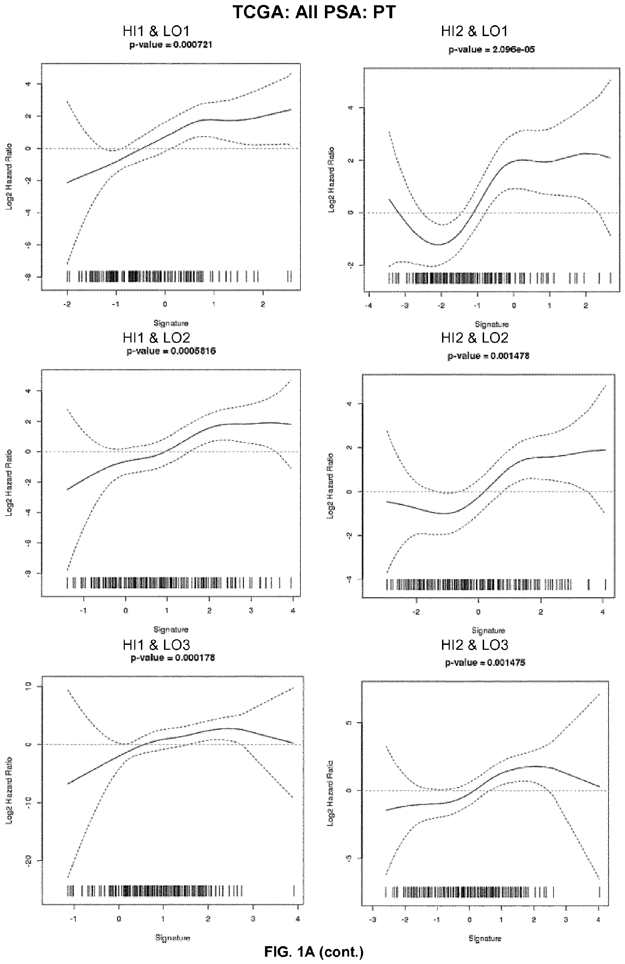

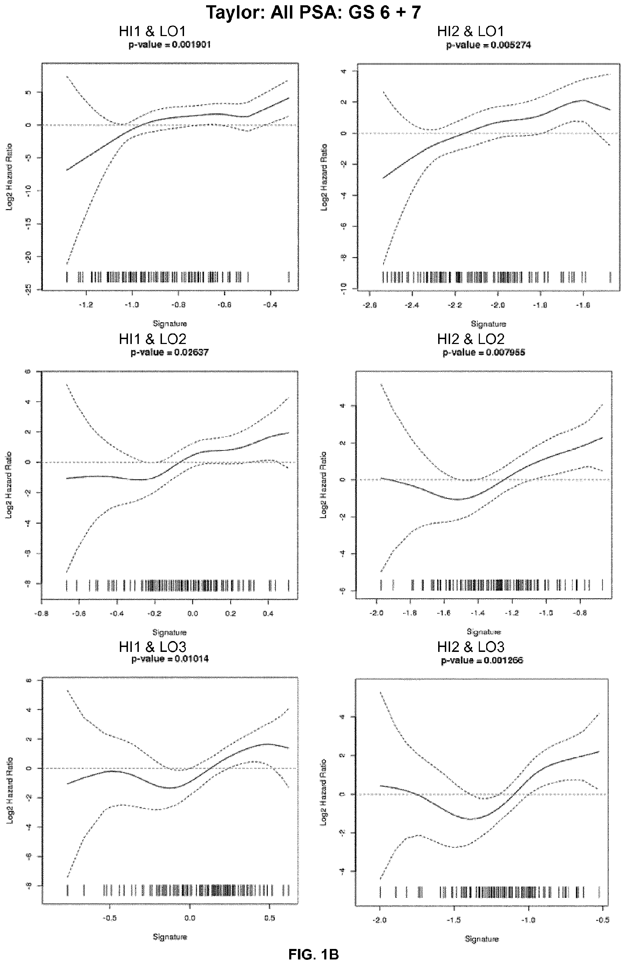

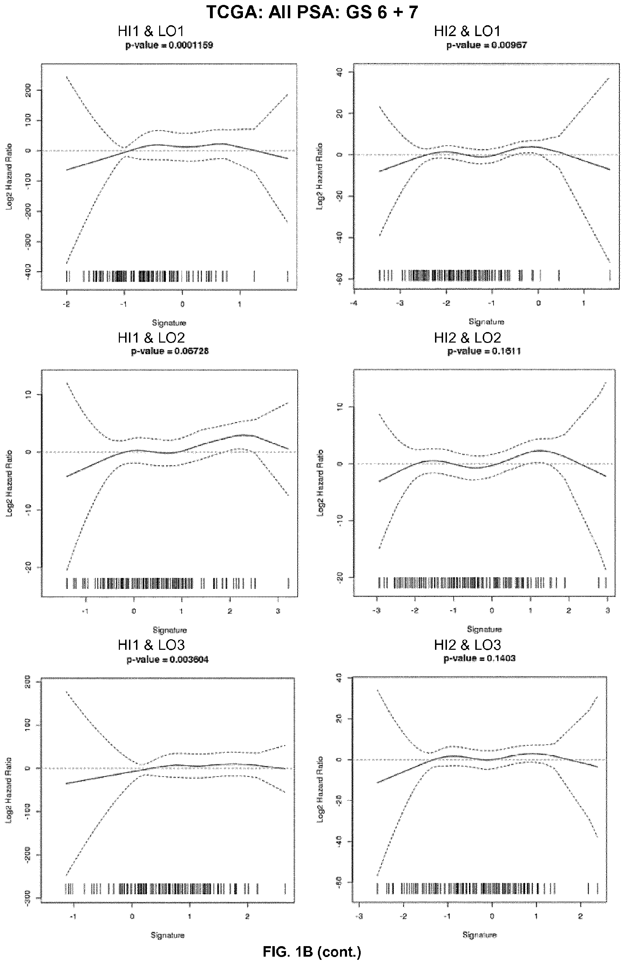

**Figure 1:** Smooth Hard Ratio (SHR) curves for all PSA prostate tumour (PT) patients (A) or for all PSA Gleason Score GS6 and GS7 patients (B). The SHR analysis relates risk of recurrence (Y axis) with the ratio between the average expression levels of two specific gene signatures (X axis). Each black line above the X axis represents one patient. The solid continuous line shows a mathematical function that relates the risk of recurrence with the average expression while dotted lines are the confidence interval. In the signatures of the invention, the calculated function shows a direct relation in which the higher the gene expression ratio, the higher risk. The small confidence interval confirms the reliability of these signatures.

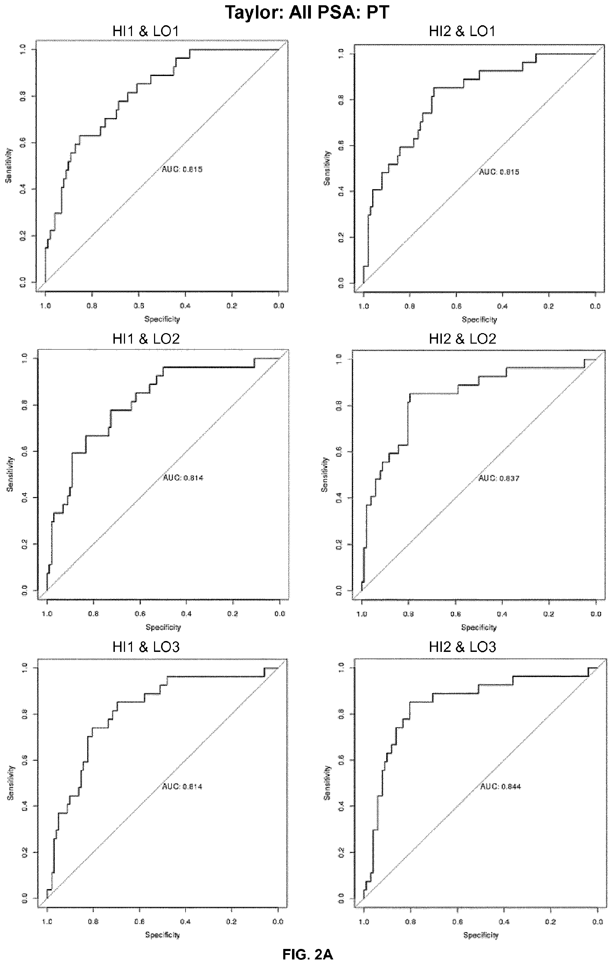

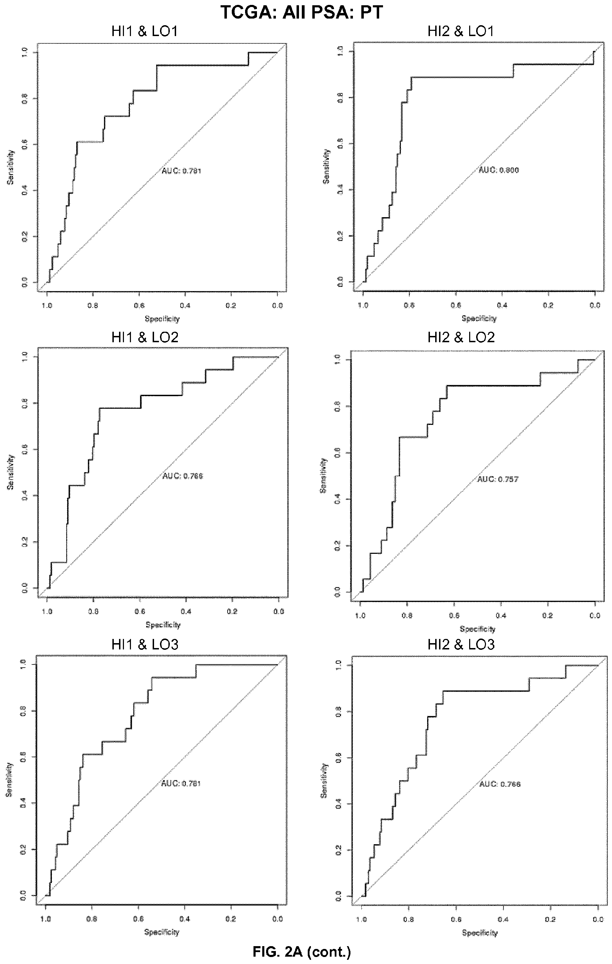

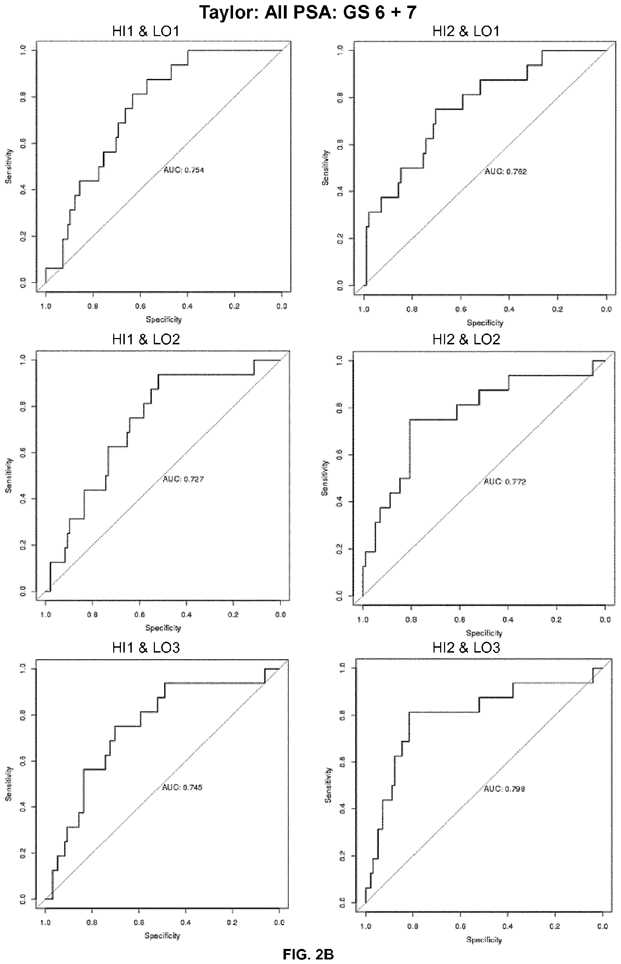

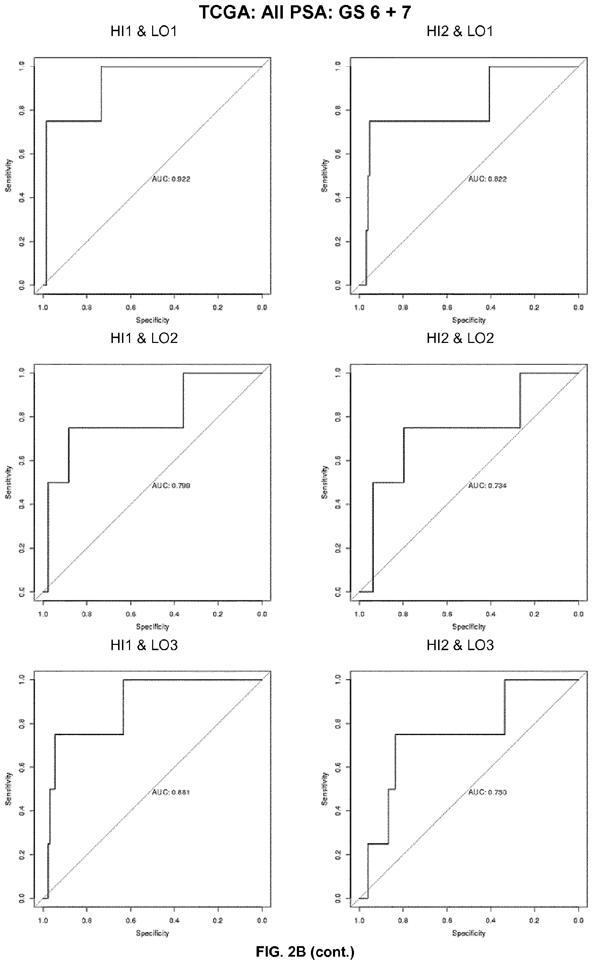

**Figure 2:** Receiver Operating Characteristic (ROC) curves for all PSA patients (A) or for Gleason Score GS6 and GS7 patients (B). In a ROC curve, true positive cases (sensitivity) are plotted against false positive (100% specificity), so that sensitivity and specificity of each signature can be obtained for predicting recurrence in a population. A test with perfect discrimination (no overlap in the two distributions) has a ROC curve that passes through the upper left corner (100% sensitivity, 100% specificity). Therefore, the closer the ROC curve is to the upper left corner, the higher the overall accuracy of the test (Zweig & Campbell, Receiver-operating characteristic (ROC) plots: a fundamental evaluation tool in clinical medicine. Clin Chem. 1993 Apr; 39(4):561-77). The minimum value of the area under the curve (AUC) considered is good in a signature used for prediction, has to be above 0.7. All AUC values obtained for the signatures of the invention are higher than 0.7. Therefore, these signatures are good predictors of recurrence.

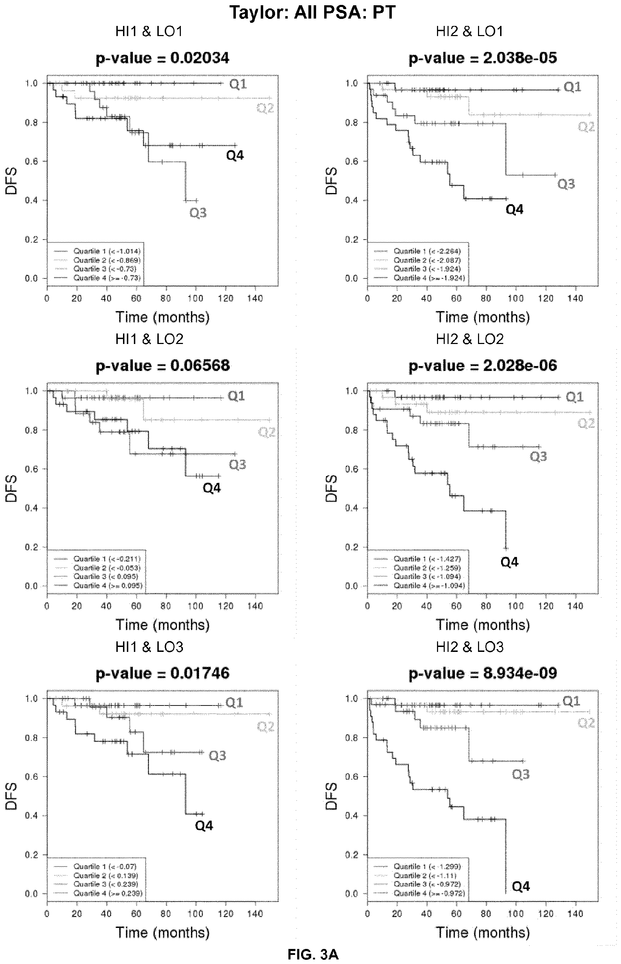

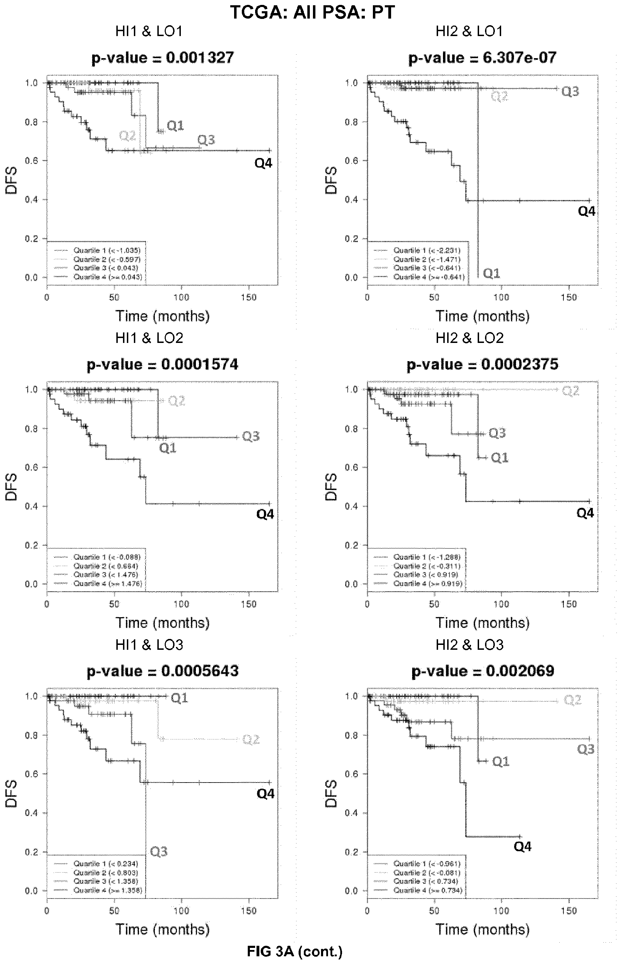

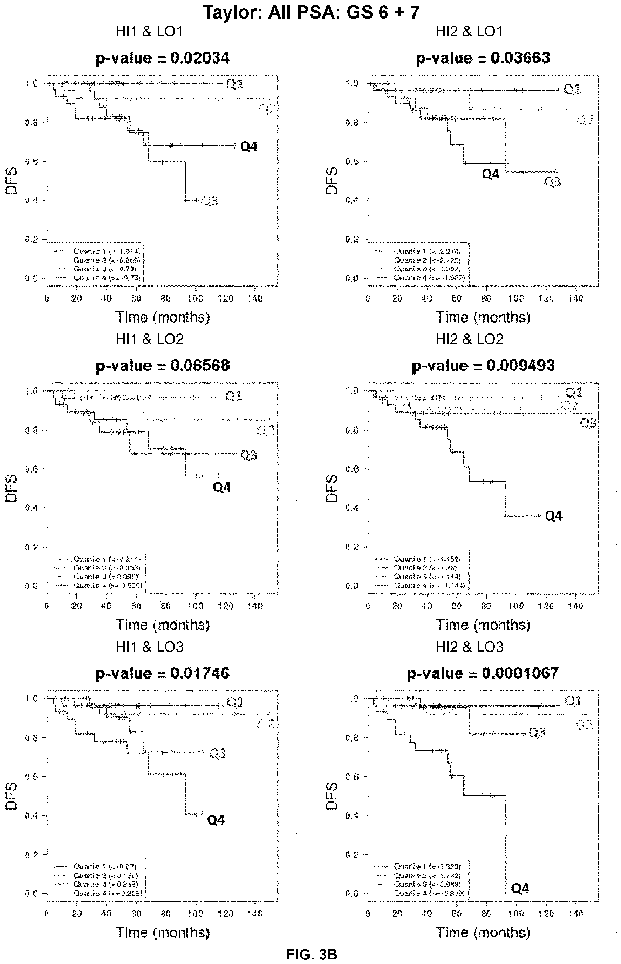

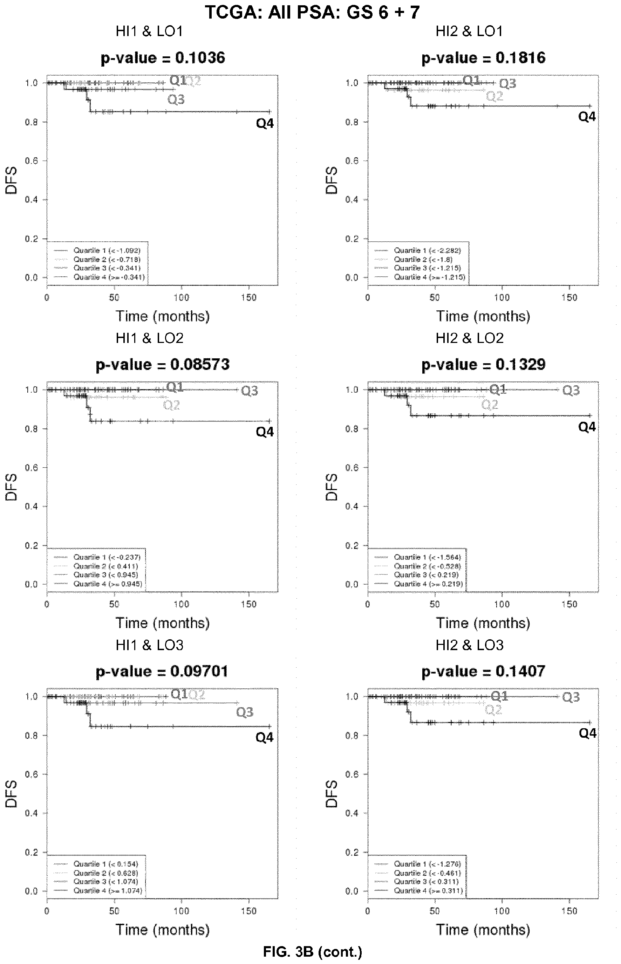

**Figure 3:** Kaplan Meier analysis for all PSA patients (A) or for Gleason Score GS6 and GS7 patients (B). This type of analysis shows the differences into the relapse of the disease among different subgroups of the population. These subgroups are obtained by separating and comparing the patients in the four different quartiles. This type of analysis is used to estimate survival and presents survival data. It shows the differences in the relapse of the disease among different subgroups of the population with time. These subgroups are obtained by separating and comparing the patients in the four different quartiles according to the gene expression ration being quartile 1 (Q1) the lowest gene expression ratio, and quartile 4 (Q4) the highest ratio. Graphics show the stratification of the different populations made by our signatures. In these results, it can be seen that the rate of survival decreases with higher gene expression ratios.

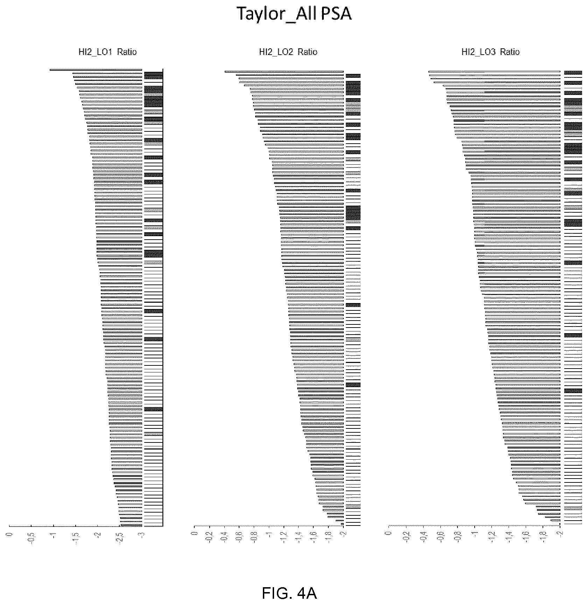

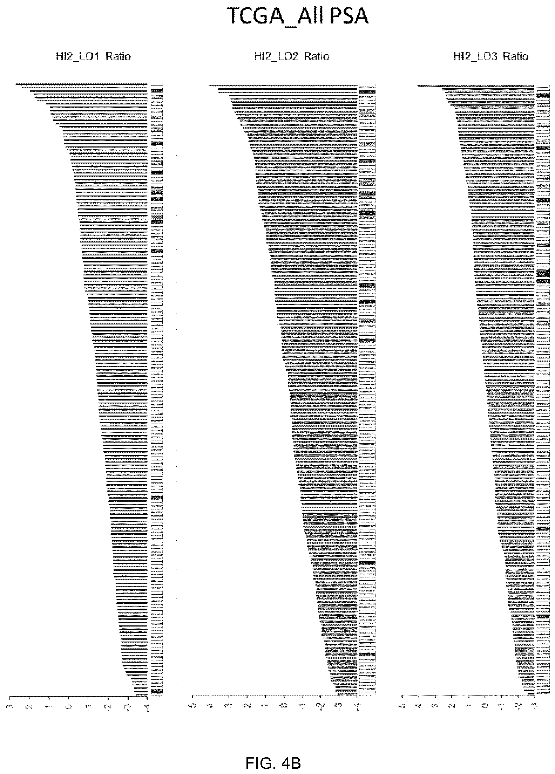

**Figure 4:** Heat maps for all PSA patients in the Taylor dataset (A) and in the TCGA dataset (B). The heat maps represent the ratio between the average expression levels of two specific gene signatures. Higher ratios represent a higher risk of recurrence (on the top of the graphic). Black lines are recurrence patients with low PSA. Grey lines are recurrence patients with high PSA.

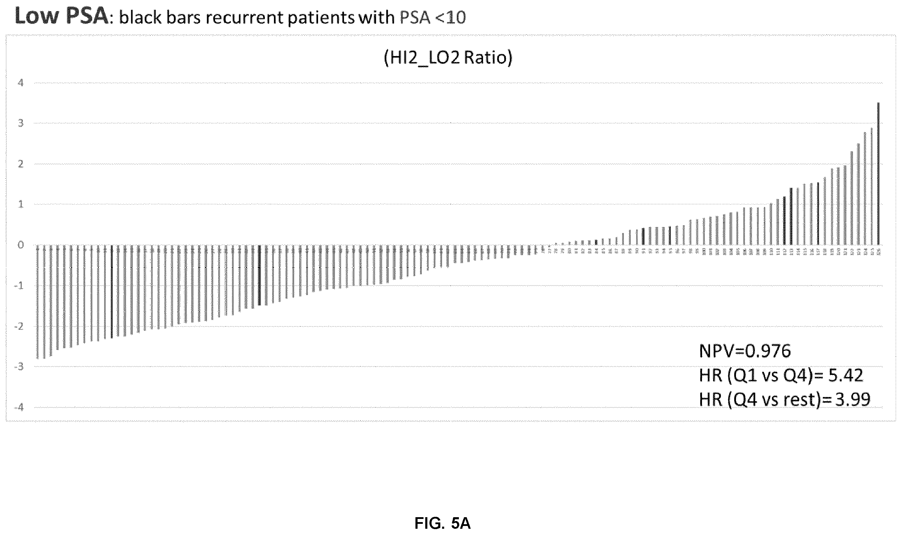

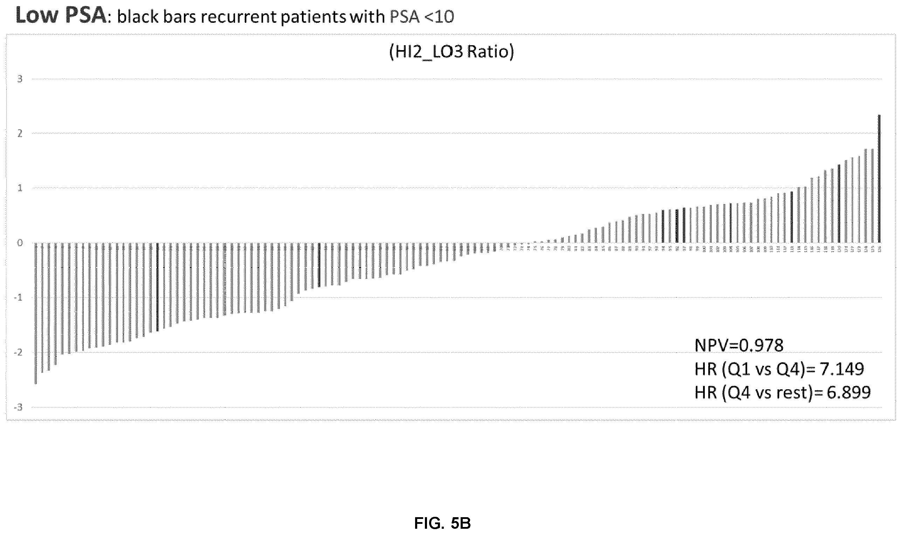

**Figure 5:** Heat maps for low PSA patients corresponding to the ratio between signatures HI2 and LO2 (A) and to the ratio between signatures HI2 and LO3 (B). Black bars represent recurrent patients with low PSA (PSA<10).

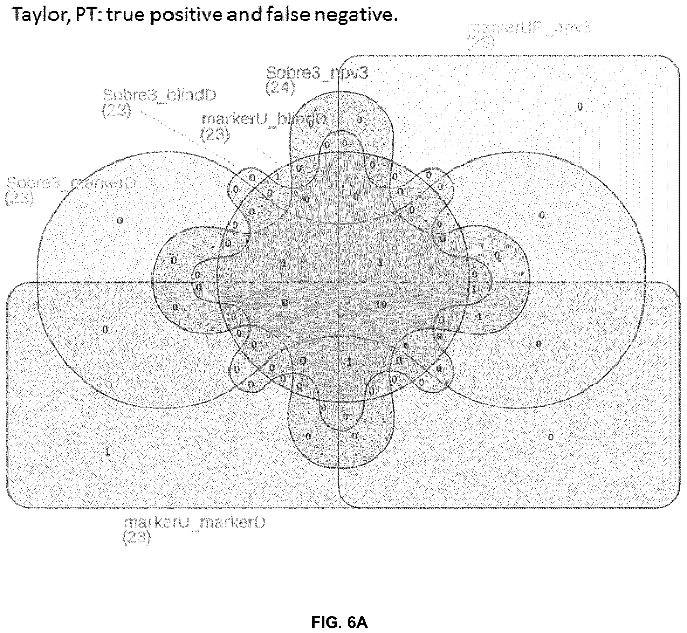

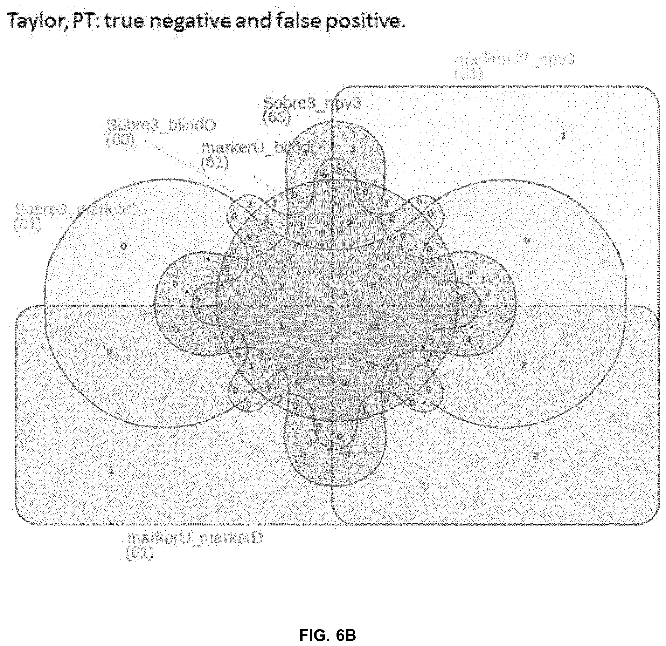

**Figure 6:** (A) True positive and false negative results for all signature ratios in the Taylor dataset, Of 27 patients who show recurrence in prostate tumour (PT), 26 are detected by at least one signature ratio, while 19 are detected in all signature ratios. (B) True negative and false positive results for all signature ratios in the Taylor dataset. Of 104 patients who show no recurrence in prostate tumour (PT), 80 are detected by at least one signature ratio, while 38 are detected in all signature ratios. In these figures, markerU_blindD is HI1_LO1, markerU_markerD is HI1_LO2, markerU_npv3 is HI1_LO3, Sobre3_blindD is HI2_LO1, Sobre3_markerD is HI2_LO2, Sobre3_npv3 is HI2_LO3.

## DETAILED DESCRIPTION OF THE INVENTION

<u>Methods for predicting the outcome of a patient suffering from prostate cancer</u>

The inventors have identified that the ratios of the average expression levels of several gene signatures are reliable markers for predicting the outcome of a patient suffering from prostate cancer.

Thus, in a first aspect, the invention relates to a method for predicting the outcome of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;

(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of an increased risk of a negative outcome for the patient, or

(ii) a decreased ratio between the average expression levels of the first gene signature and the average expression levels of the second gene signature with respect to a reference value is indicative of a decreased risk of a negative outcome for the patient.

In a particular embodiment, the first gene signature of the invention is HI1 (CPOX, GAL3St4, ORAI2 and NIPA1). In a particular embodiment, the first gene signature of the invention is HI2 (KHK, NOX4, PTGES3 and RRM2). In a particular embodiment, the second gene signature of the invention is LO1 (ATP8B1, CDO1, CHRNA2, GLB1L3, GNE, KATNAL2, PTGIS, SLC6A14, TP53INP2, and TRIM47). In a particular embodiment, the second gene signature of the invention is LO2 (ASPA, CDO1, CYP3A5, GLB1L2, GNE, ITPKC, KCTD14, PAH, PHYHD1, SIRT1, SLC40A1, SLC7A4, and SRD5A). In a particular embodiment, the second gene signature of the invention is LO3 (ASPA, CDO1, GFPT2, GNE, ITPKC, PAH, SIRT1 and TPMT).

In a particular embodiment, the ratio between the average expression levels of a first gene signature and the average expression levels of a second gene signature is the ratio between HI1 (CPOX, GAL3St4, ORAI2 and NIPA1) and LO1 (ATP8B1, CDO1, CHRNA2, GLB1L3, GNE, KATNAL2, PTGIS, SLC6A14, TP53INP2, and TRIM47). In a particular embodiment, the ratio between the average expression levels of a first gene signature and the average expression levels of a second gene signature is the ratio between HI1 (CPOX, GAL3St4, ORAI2 and NIPA1)and LO2 (ASPA, CDO1, CYP3A5, GLB1L2, GNE, ITPKC, KCTD14, PAH, PHYHD1, SIRT1, SLC40A1, SLC7A4, and SRD5A). In a particular embodiment, the ratio between the average expression levels of a first gene signature and the average expression levels of a second gene signature is the ratio between HI1 (CPOX, GAL3St4, ORAI2 and NIPA1) and LO3 (ASPA, CDO1, GFPT2, GNE, ITPKC, PAH, SIRT1 and TPMT).

In a particular embodiment, the ratio between the average expression levels of a first gene signature and the average expression levels of a second gene signature is the ratio between HI2 (KHK, NOX4, PTGES3 and RRM2) and LO1 (ATP8B1, CDO1, CHRNA2, GLB1L3, GNE, KATNAL2, PTGIS, SLC6A14, TP53INP2, and TRIM47). In a particular embodiment, the ratio between the average expression levels of a first gene signature and the average expression levels of a second gene signature is the ratio between HI2 (KHK, NOX4, PTGES3 and RRM2) and LO2 (ASPA, CDO1, CYP3A5, GLB1L2, GNE, ITPKC, KCTD14, PAH, PHYHD1, SIRT1, SLC40A1, SLC7A4, and SRD5A). In a particular embodiment, the ratio between the average expression levels of a first gene signature and the average expression levels of a second gene signature is the ratio between HI2 (KHK, NOX4, PTGES3 and RRM2) and LO3 (ASPA, CDO1, GFPT2, GNE, ITPKC, PAH, SIRT1 and TPMT).

In a particular embodiment, all of gene signature ratios H1_LO1; H1_LO2; H1_LO3; H2_LO1; H2_LO2 and H2_LO3 are determined for the patient.

The term "ASPA", also known as ACY2, ASP, as used herein refers to the human gene corresponding with the Ensembl database accession number ENSG00000108381.

The term "ATP8B1", also known as ATPIC, BRIC, FIC1, ICP1, PFIC, PFIC1, as used herein refers to the human gene corresponding with the Ensembl database accession number ENSG00000081923.

The term "CDO1", also known as CDO-I, as used herein refers to the human gene corresponding with the Ensembl database accession number ENSG00000129596.

The term "CHRNA2" as used herein refers to the human gene corresponding with the Ensembl database accession number ENSG00000120903.

The term "CPOX", also known as CPO, CPX, HCP, as used herein refers to the human gene corresponding with the Ensembl database accession number ENSG00000080819.

The term "CYP3A5", also known as CP35, CYPIIIA5, P450PCN3, PCN3, as used herein refers to the human gene corresponding with the Ensembl database accession number ENSG00000106258.

The term "GAL3ST4", also known as GAL3ST-4, as used herein refers to the human gene corresponding with the Ensembl database accession number ENSG00000197093.

The term "GFPT2", also known as GFAT, GFAT 2, GFAT2, as used herein refers to the human gene corresponding with the Ensembl database accession number ENSG00000131459.

The term "GLB1L2", also known as MST114, MSTP114, as used herein refers to the human gene corresponding with the Ensembl database accession number ENSG00000149328.

The term "GLB1L3", as used herein refers to the human gene corresponding with the Ensembl database accession number ENSG00000166105.

The term "GNE", also known as DMRV, GLCNE, IBM2, NM, Uae1, as used herein refers to the human gene corresponding with the Ensembl database accession number ENSG00000159921.

The term "ITPKC", also known as IP3-3KC, IP3KC, as used herein refers to the human gene corresponding with the Ensembl database accession number ENSG00000086544.

The term "KATNAL2" as used herein refers to the human gene corresponding with the Ensembl database accession number ENSG00000167216.

The term "KCTD14" as used herein refers to the human gene corresponding with the Ensembl database accession number ENSG00000151364.

The term "KHK" as used herein refers to the human gene corresponding with the Ensembl database accession number ENSG00000138030.

The term "NOX4", also known as KOX, KOX-1, RENOX, as used herein refers to the human gene corresponding with the Ensembl database accession number ENSG00000086991.

The term "ORAI2", also known as C7orf19, CBCIP2, MEM142B, TMEM142B, as used herein refers to the human gene corresponding with the Ensembl database accession number ENSG00000160991.

The term "PAH", also known as PH, PKU, PKU1, as used herein refers to the human gene corresponding with the Ensembl database accession number ENSG00000171759.

The term "PHYHD1" as used herein refers to the human gene corresponding with the Ensembl database accession number ENSG00000175287.

The term "PTGES3", also known as P23, TEBP, cPGES, as used herein refers to the human gene corresponding with the Ensembl database accession number ENSG00000110958.

The term "PTGIS", also known as CYP8, CYP8A1, PGIS, PTGI, as used herein refers to the human gene corresponding with the Ensembl database accession number ENSG00000124212.

The term "RRM2", also known as R2, RR2, RR2M, as used herein refers to the human gene corresponding with the Ensembl database accession number ENSG00000171848.

The term "SIRT1", also known as SIR2, SIR2L1, SIR2alpha, as used herein refers to the human gene corresponding with the Ensembl database accession number ENSG00000096717.

The term "SLC40A1", also known as FPN1, HFE4, IREG1, MST079, MSTP079, MTP1, SLC11A3, as used herein refers to the human gene corresponding with the Ensembl database accession number ENSG00000138449.

The term "SLC6A14", also known as BMIQ11, as used herein refers to the human gene corresponding with the Ensembl database accession number ENSG00000268104.

The term "SLC7A4", also known as CAT-4, CAT4, HCAT3, VH, as used herein refers to the human gene corresponding with the Ensembl database accession number ENSG00000099960.

The term "SRD5A2" as used herein refers to the human gene corresponding with the Ensembl database accession number ENSG00000277893.

The term "TP53INP2", also known as C20orf110, DOR, PIG-U, PIGU, PINH, dJ1181N3.1, as used herein refers to the human gene corresponding with the Ensembl database accession number ENSG00000078804.

The term "TPMT", also known as TPMTD, as used herein refers to the human gene corresponding with the Ensembl database accession number ENSG00000137364.

The term "TRIM47", also known as GOA, RNF100, as used herein refers to the human gene corresponding with the Ensembl database accession number ENSG00000132481.

In a particular embodiment, the reference value is the mean ratio between the average expression levels of the first gene signature and the average expression levels of the second gene signature in a pool of samples from primary prostate tumours,

(a) wherein the first gene signature is selected from the group consisting of HI1 and HI2, wherein gene signature HI1 consists of genes CPOX, GAL3St4, ORAI2 and NIPA1; and wherein HI2 consists of genes KHK, NOX4, PTGES3 and RRM2;

(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

"Reference value", as used herein in the context of the first method of the invention, refers to a laboratory value used as a reference for values/data obtained by laboratory examinations of subjects or samples collected from patients. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time or from a non-cancerous tissue. The reference value can be based on a large number of samples, such as from population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

In a preferred embodiment, the "average expression" of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes); or LO3 (i.e., the ASPA, CDO1, GFPT2, GNE, ITPKC, PAH, SIRT1 and TPMT genes) may be obtained by first determining the individual expression level for each gene and then calculating the average of the individual expression levels for all genes. In a different embodiment, the "average expression" of the genes may be directly obtained by determining the expression level for all genes. In this embodiment, individual expression levels for each gene are not calculated. Since all genes have the same directionality in their altered condition (i.e. they are all upregulated in HI1 and HI2, and they are all downregulated in LO1, LO2 and LO3), the average signal can be taken to inform on the activity of the cellular energy metabolism pathway.

In a preferred embodiment, the reference value is the expression levels of the gene of interest in a pool obtained from primary prostate tumour tissues obtained from patients. This pool will include patients with good prognosis and patients with bad prognosis and therefore, the expression levels would be an average value of the values found in the different types of patients.

In another embodiment, the reference value is the expression levels of the gene of interest in primary prostate tumour tissue obtained from a patient or patients identified as patients having a good prognosis. In another embodiment, the reference value is the expression levels of the gene of interest in a tumour tissue obtained from a patient or patients identified as patients having a bad prognosis.

In case that the reference value is the expression level of the gene of interest obtained from primary prostate tumour tissues from patients having a good prognosis, then patients can be identified as having poor prognosis if the expression levels are lower than the reference value. In case that the reference value is the expression level of the gene of interest obtained from tumour tissues from patients having a poor prognosis, then patients can be identified as having good prognosis if the expression levels are higher than the reference value.

The sample collection from which the reference level is derived will preferably be formed by subjects suffering from the same type of cancer as the patient object of the study.

Moreover, a reference value has to be established for each gene to be measured. In another embodiment, the quantity of any one or more biomarkers in a sample from a tested subject may be determined directly relative to the reference value (e.g., in terms of increase or decrease, or fold-increase or fold-decrease). Advantageously, this may allow to compare the quantity of any one or more biomarkers in the sample from the subject with the reference value (in other words to measure the relative quantity of any one or more biomarkers in the sample from the subject vis-a-vis the reference value) without the need to first determine the respective absolute quantities of said one or more biomarkers.

Once this reference value is established, the level of this marker expressed in tumour tissues from subjects can be compared with this reference value, and thus be assigned a level of "increased" or "decreased". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

The comparison of the expression levels of the gene of interest with the reference value allows determining whether the patient will show a good or poor prognosis. In the first method of the invention, an increase in the ratio between the average expression levels of the first gene signature and the average expression levels of the second gene signature with respect to a reference value is indicative of an increased risk of a negative outcome for the patient, whereas a decrease in the ratio between the average expression levels of the first gene signature and the average expression levels of the second gene signature with respect to a reference value is indicative of a decreased risk of a negative outcome for the patient.

As used herein, "a decreased risk of negative outcome" indicates that the subject is expected (e.g. predicted) to survive and/or have no, or is at low risk of having, recurrence or distant metastases within a set time period. The term "low" is a relative term and, in the context of this application, refers to the risk of the "low" expression group with respect to a clinical outcome (recurrence, distant metastases, etc.). A "low" risk can be considered as a risk lower than the average risk for a heterogeneous cancer patient population. In the study of Paik et al. (2004), an overall "low" risk of recurrence was considered to be lower than 15 percent. The risk will also vary in function of the time period. The time period can be, for example, five years, ten years, fifteen years or even twenty years after initial diagnosis of cancer or after the prognosis is made.

As used herein, "an increased risk of negative outcome" indicates that the subject is expected, i.e. predicted, to not survive and/or to have, or is at high risk of having, recurrence or distant metastases within a set time period. The term "high" is a relative term and, in the context of this application, refers to the risk of the "high" expression group with respect to a clinical outcome (recurrence, distant metastases, etc.). A "high" risk can be considered as a risk higher than the average risk for a heterogeneous cancer patient population. The risk will also vary in function of the time period. The time period can be, for example, five years, ten years, fifteen years or even twenty years of initial diagnosis of cancer or after the prognosis was made.

As used herein, the term "gene signature" refers to a set of one or more differentially expressed genes that are statistically significant and characteristic of the biological differences between two or more cell samples, e.g., control subjects or prostate cancer patients with increased risk of negative outcome. A signature may be expressed as a number of individual unique probes complementary to signature genes whose expression is detected when a cRNA product is used in microarray analysis or in a PCR reaction. A signature may be exemplified by a particular set of genes making up a biomarker.

As used herein, the expression "average expression level" is understood as arithmetic average of logarithm-transformed values of gene expression levels, in whatever units are chosen, as measured on any applicable platform, as listed above. The expression average should be understood in the wide sense as covering not only the arithmetic mean but also other calculation methods, such as the geometric mean.

The term "outcome" refers to a prediction of medical prognosis, for example, a poor or good outcome (e.g., likelihood of long-term survival, overall survival, disease-specific survival, progression-free survival or disease-free survival); a negative prognosis, or poor outcome, includes a prediction of relapse, disease progression (e.g., tumour growth or metastasis, or drug resistance), or mortality; whereas a positive prognosis, or good outcome, includes a prediction of disease remission, (e.g., disease-free status), amelioration (e.g., tumour regression), or stabilization.

Any parameter which is widely accepted for determining the outcome of a patient can be used in the present invention including, without limitation:

- overall survival rate, as used herewith, relates to the percentage of people in a study or treatment group who are alive for a certain period of time after they were diagnosed with or treated for a disease, such as cancer.
- disease-specific survival rate, which is defined as the percentage of people in a study or treatment group who have not died from a specific disease in a defined period of time.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumour control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- time to progression (TTP), as used herein, relates to the time since a disease is treated until the disease starts to get worse.
- six-month progression free survival or "PFS6" rate which, as used herein, relates to the percentage of subjects who are free of progression in the first six months after the initiation of the therapy and
- median survival which, as used herein, relates to the time at which half of the subjects enrolled in the study are still alive.

In a particular embodiment, the outcome of the patient is determined as time to cancer recurrence, overall survival, disease-specific survival, disease-free survival or occurrence of metastasis.

The term "patient" or "subject" refers to a human male of any age or race. In a preferred embodiment, the subject has not been treated with chemotherapy or radiotherapy prior to the determination of the expression levels of the gene or genes of interest. In yet another embodiment, the patient has undergone surgical resection of the tumour. In a particular embodiment, the patient is a patient in which a primary prostate tumour has been surgically excised.

As used herein, "sample" or "biological sample" means biological material isolated from a subject. The biological sample may contain any biological material suitable for determining the expression level of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes); and/or LO3 (i.e., the ASPA, CDO1, GFPT2, GNE, ITPKC, PAH, SIRT1 and TPMT genes). The sample can be isolated from any suitable biological tissue or fluid such as, for example, tumour tissue, blood, plasma, serum, sputum, broncho-alveolar lavage, urine or cerebral spinal fluid (CSF). In a preferred embodiment, the sample contains tumour cells. In a more preferred embodiment, the sample containing tumour cells is a tumour tissue sample. The tumour tissue sample is understood as the tissue sample originating from the primary tumour or from a distant metastasis. Said sample can be obtained by conventional methods, for example biopsy, using methods well known by the person skilled in related medical techniques. Alternatively, the tumour tissue sample may be a sample of a tumour which has been surgically resected. The methods for obtaining a biopsy sample include splitting a tumour into large pieces, or microdissection, or other cell separating methods known in the art. The tumour cells can additionally be obtained by means of cytology through aspiration with a small gauge needle. To simplify sample preservation and handling, samples can be fixed in formalin and soaked in paraffin or first frozen and then soaked in a tissue freezing medium such as OCT compound by means of immersion in a highly cryogenic medium which allows rapid freezing. In a preferred embodiment, the tumour tissue is a tumour biopsy or a surgically resected tumour.

The term "expression level" of a gene as used herein refers to the measurable quantity of gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene.

The expression levels of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes); and/or LO3 (i.e., the ASPA, CDO1, GFPT2, GNE, ITPKC, PAH, SIRT1 and TPMT genes) can be determined by measuring the levels of mRNA encoded by said genes, or by measuring the levels of the protein encoded by said genes, i.e. the KHK, NOX4, PTGES3 and RRM2 proteins; the CPOX, GAL3St4, ORAI2 and NIPA1 proteins; the ASPA, CDO1, CYP3A5, GLB1L2, GNE, ITPKC, KCTD14, PAH, PHYHD1, SIRT1, SLC40A1, SLC7A4, and SRD5A proteins; the ASPA, CDO1, GFPT2, GNE, ITPKC, PAH, SIRT1 and TPMT proteins; or theATP8B1, CDO1, CHRNA2, GLB1L3, GNE, KATNAL2, PTGIS, SLC6A14, TP53INP2, and TRIM47 proteins, or of variants thereof. In a particular embodiment, the determination of the expression levels of the genes in the gene signatures is carried out by determining the levels of the corresponding mRNAs or by determining the levels of the polypeptides encoded by said genes.

In order to measure the mRNA levels of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and/or LO3 (i.e., the ASPA, CDO1, GFPT2, GNE, ITPKC, PAH, SIRT1 and TPMT genes), the biological sample may be treated to physically, mechanically or chemically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumour tissue such as a resected tumour.

In a preferred embodiment samples can be obtained from fresh tumour tissue or from OCT embedded frozen tissue. In another preferred embodiment samples can be obtained by bronchoscopy and then paraffin-embedded.

Determination of the levels of mRNA of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and/ or LO3 (i.e., the ASPA, CDO1, GFPT2, GNE, ITPKC, PAH, SIRT1 and TPMT genes) can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the levels of the above genes can also be carried out by Fluorescence In Situ Hybridization, including variants such as Flow-FISH, qFiSH and double fusion FISH (D-FISH) as described in WO2010030818. The levels of the mRNA of the different genes can also be determined by nucleic acid sequence based amplification (NASBA) technology. In a preferred embodiment, the gene mRNA expression levels are often determined by reverse transcription polymerase chain reaction (RT-PCR). Thus, in a particular embodiment, the mRNA expression levels of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes); and/or LO3 (i.e., the ASPA, CDO1, GFPT2, GNE, ITPKC, PAH, SIRT1 and TPMT genes) are determined by quantitative PCR, preferably, Real-Time PCR. The detection can be carried out in individual samples or in tissue microarrays. In a particular embodiment, the mRNA expression levels are determined by molecular barcoding technology (i.e. Nanostring technology). In a particular embodiment, the mRNA expression levels are determined by sequencing, preferably High-Throughput Sequencing (Jason A. Reuter et al., High-Throughput Sequencing Technologies, Mol Cell. 2015 May 21; 58(4): 586-597).

Alternatively, in another embodiment, the expression level of HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and/or LO3 (i.e., the ASPA, CDO1, GFPT2, GNE, ITPKC, PAH, SIRT1 and TPMT genes) is determined by measuring the expression of the polypeptides encoded by said genes or of variants thereof. In a preferred embodiment the expression level of the protein or of variants thereof is determined by Western blot, ELISA or by immunohistochemistry.

The expression levels of the proteins encoded by HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and/or LO3 (i.e., the ASPA, CDO1, GFPT2, GNE, ITPKC, PAH, SIRT1 and TPMT genes) can be quantified by means of conventional methods, for example, using antibodies with a capacity to specifically bind to the proteins encoded by said genes(or to fragments thereof containing antigenic determinants) and subsequent quantification of the resulting antibody-antigen complexes.

The antibodies to be employed in these assays can be, for example, polyclonal sera, hybridoma supernatants or monoclonal antibodies, antibody fragments, Fv, Fab, Fab' and F(ab')$_2$, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

Alternatively, in another particular embodiment, the levels of the proteins encoded by HI1 (i.e., the CPOX, GAL3St4, ORAI2 and NIPA1 genes); HI2 (i.e., the KHK, NOX4, PTGES3 and RRM2 genes); LO1 (i.e., the ATP8B1, CDO1, CHRNA2, GLB1L3, GNE, KATNAL2, PTGIS, SLC6A14, TP53INP2, and TRIM47 genes); LO2 (i.e., the ASPA, CDO1, CYP3A5, GLB1L2, GNE, ITPKC, KCTD14, PAH, PHYHD1, SIRT1, SLC40A1, SLC7A4, and SRD5A genes) and/or LO3 (i.e., the ASPA, CDO1, GFPT2, GNE, ITPKC, PAH, SIRT1 and TPMT genes) or of the variants thereof are determined by Western blot. Western blot is based on the detection of proteins previously resolved by gel electrophoreses under denaturing conditions and immobilized on a membrane, generally nitrocellulose, by the incubation with an antibody specific and a developing system (e.g. chemoluminiscent).

In a particular embodiment, the first method of the invention further comprises the determination of one or more clinical parameters which are also indicative of the prognosis of the cancer. Such indicators include the presence or levels of known cancer markers, or can be clinical or pathological indicators (for example, age, tumour size, tumour histology, differentiation grade, clinical stage, family history and the like). In a preferred embodiment, the method of the invention further comprises the determination of the Prostate Specific Antigen (PSA).

The term "PSA", also known as KLK3, kallikrein related peptidase 3, APS, as used herein refers to the human gene corresponding with the Ensembl database accession number ENSG00000142515. Men with prostate cancer may be characterized as low-, intermediate-, or high-risk for having/developing metastatic disease or dying of prostate cancer. PSA level is one of three variables on which the risk-stratification is based; the others are the grade of prostate cancer (Gleason grading system) and the stage of cancer based on physical examination and imaging studies. D'Amico Criteria for each risk category are as follows (D'Amico A.V., et al. (1998). "Biochemical outcome after radical prostatectomy, external beam radiation therapy, or interstitial radiation therapy for clinically localized prostate cancer". JAMA. 280 (11): 969-74):

- Low-risk: PSA < 10, Gleason score $\leq$ 6, AND clinical stage $\leq$ T2a;
- Intermediate-risk: PSA 10-20, Gleason score 7, OR clinical stage T2b/c;
- High-risk: PSA > 20, Gleason score $\geq$ 8, OR clinical stage $\geq$ T3.

The person skilled in the art will understand that the determination of the prognosis is not needed to be correct for all the subjects (i.e., for 100% of the subjects). Nevertheless, the term requires enabling the identification of a statistically significant part of the subjects (for example, a cohort in a cohort study). Whether a part is statistically significant can be determined in a simple manner by the person skilled in the art using various well known statistical evaluation tools, for example, the determination of confidence intervals, determination of p values, Student's T test, Mann-Whitney test, etc. The preferred confidence intervals are at least 90%, at least 95%, at least 97%, at least 98% or at least 99%.

The p values are preferably 0.1, 0.05, 0.01, 0.005 or 0.0001. More preferably, at least 60%, at least 70%, at least 80% or at least 90% of the subjects of a population can be suitably identified by the method of the present invention.

All the terms and embodiments described elsewhere herein are equally applicable to this aspect of the invention.

Method for predicting the response to therapy of a patient suffering from prostate cancer

In a second aspect, the invention also relates to a method for predicting response to therapy of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein gene signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of a negative response of the patient to the therapy, or
(ii) a decreased ratio between the average expression levels of the first gene signature and the average expression levels of the second gene signature with respect to a reference value is indicative of a positive response of the patient to the therapy.

The expression "positive response" when referred to the treatment for advanced, recurrent or metastatic prostate cancer, as used herein, refers to any response which is substantially better than that obtained with a saline control or placebo. The response can be assessed using any endpoint indicating a benefit to the patient, including, without limitation, (1) inhibition, to some extent, of tumour growth, including slowing down and complete growth arrest; (2) reduction in the number of tumour cells; (3) reduction in tumour size; (4) inhibition (i.e., reduction, slowing down or complete stopping) of tumour cell infiltration into adjacent peripheral organs and/or tissues; (5) inhibition of metastasis; (6) enhancement of anti-tumour immune response, possibly resulting in regression or rejection of the tumour; (7) relief, to some extent, of one or more symptoms associated with the tumour; (8) increase in the length of survival following treatment; and/or (9) decreased mortality at a given point of time following treatment. Positive clinical response may also be expressed in terms of various measures of clinical outcome as defined elsewhere herein. Positive clinical outcome can also be considered in the context of an individual's outcome relative to an outcome of a population of patients having a comparable clinical diagnosis, and can be assessed using various endpoints such as an increase in the duration of Recurrence-Free interval (RFI), an increase in the time of survival as compared to Overall Survival (OS) in a population, an increase in the time of Disease-Free Survival (DFS), an increase in the duration of Distant Recurrence-Free Interval (DRFI), and the like. An increase in the likelihood of positive clinical response corresponds to a decrease in the likelihood of cancer recurrence.

As used herein the term "a negative response" when referred to the treatment for advanced, recurrent or metastatic prostate cancer means that the treatment provides no reduction of the assessed symptoms of the cancer or causes an increase in the symptoms of the cancer.

All the terms and embodiments described elsewhere herein are equally applicable to this aspect of the invention.

Method for selecting a patient to be treated for prostate cancer

In a third aspect, the invention relates to a method for selecting a patient to be treated for low risk, indolent, localised, advanced, recurrent or metastatic prostate cancer which comprises determining, in a sample from the patient, the ratio between the average expression levels of a first gene signature and the average expression levels of a second gene signature,

(a) wherein the first gene signature is selected from the group consisting of HI1 and HI2, wherein signature HI1 consists of genes CPOX, GAL3St4, ORAI2, NIPA1; and wherein signature HI2 consists of genes KHK, NOX4, PTGES3 and RRM2;

(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein an increased ratio between the average expression levels of the first gene signature and the average expression levels of the second gene signature with respect to a reference value is indicative that the patient is selected for treatment for prostate cancer.

The term "prostate cancer", as used herein, refers to any cancer of the prostate gland in which cells of the prostate mutate and begin to multiply out of control irrespective of the stage of the cancer. The extent to which prostate cancer has progressed in a patient is assessed taking into account clinical and histopathological information. The stage of cancer is classified based on the TNM system, taking into account tumour size (T), whether there is lymph node involvement (N), the presence of metastasis (M), and the tumour grading (G). A tumour classed as T1 is confined to the prostate gland and too small to be felt by digital rectal examination. T1 further includes T1a (fewer than 5% cancerous cells in tissue sample) and T1b (more than 5%) subdivisions. Tic indicates the patient has an elevated Prostate Specific Antigen (PSA; see definition later). If the tumour is large enough to be felt during a digital rectal examination, it is classified as T2. T2a means only one side of the prostate gland (left or right) is involved; T2b means both sides have a tumour(s). T2 is commonly termed "localized cancer". If the cancer is T3, it has spread to the connected tissue near the prostate (T3a) or the seminal vesicles (T3b). T4 indicates cancer spread to tissue next to the prostate, for example the bladder sphincter, rectum or pelvis wall. The prostate cancer may also spread into the regional lymph nodes of the pelvis and this is assessed as N1 stage of prostate cancer. These stages of T3, T4 and N1 are collectively termed "locally advanced" or regional cancer. If the cancer has spread to distant sites, such as the bone, it is said to be "metastasized" or at the M1 stage. Prostate cancer that has spread to distant lymph nodes is categorized as M1 a while that which has spread to bone is M1b and that which has spread to organs such as liver or brain is assessed as M1c. If left untreated, prostate cancer almost universally metastasizes to bone, although it can also spread to liver and lungs.

Like staging, grading levels are also assigned to prostate cancer cases. Grading takes place after a biopsy (removal and examination of tissue) is done. The grade refers to the appearance of the tumour cells and indicates the rate of growing. Most pathologists grade prostate cancer according to the Gleason score, which assigns a grade from 1 to 5 based on how the cancerous cells look compared to normal prostate cells. If the cancerous cells look very similar to the healthy cells, the cancerous cells are called well-differentiated. If the cancerous cells are very different from the healthy cells, however, they are called poorly differentiated.

The pathologist studies the patterns of cancerous cells under a microscope. Based on the most common pattern of differentiation, the pathologist will assign a number 1 through 5. Then based on the second most common pattern of cell differentiation, the pathologist assigns a second number of 1 through 5. The sum of these two numbers gives the Gleason score, which can range from 2 through 10. Thus the higher the Gleason score, the more likely it is that the cancer will grow and spread rapidly. Pathologists often identify the two most common patterns of cells in the tissue, assign a Gleason grade to each, and add the two grades. Two of the common patterns of cells in the tissue for example are percentage of positive cells and the intensity of positively stained cells. The result is a number between 2 and 10. A Gleason score lower than 6 indicates a less aggressive cancer. A Gleason score of 7 and higher is considered more aggressive. According to the Gleason score, prostate cancer can be classified according to their aggressiveness as low aggressive tumours, those having a Gleason a score of 5 or below; intermediate aggressive tumours, those having a Gleason score of 6; and highly aggressive tumours, those having a Gleason score of 7 or higher, until 10, with the more aggressive forms of prostate cancer being those which have scores of 8, 9, or 10. Prostate cancer cases with a Gleason score below 4 are very rare, as they usually do not warrant the biopsy in the first place.

In a particular embodiment, the prostate cancer is low risk prostate cancer. A low risk prostate cancer refers to a prostate tumour that grows very slowly, or which does not grow at all (Informed Health Online [Internet]. What is low-risk prostate cancer and how is it treated? www.ncbi.nlm.nih.gov/books/NBK487255/). In another particular embodiment, the prostate cancer is indolent prostate cancer. Indolent prostate cancer refers to a prostate tumour that is unlikely to become symptomatic during life (Bangma CH, Roobol MJ. Defining and predicting indolent and low risk prostate cancer. Crit Rev Oncol Hematol. 2012 Aug; 83(2):235-41). In a particular embodiment, the prostate cancer is localised prostate cancer (www.ncbi.nlm.nih.gov/books/NBK248403/). Localised prostate cancer is a prostate cancer that is confined within the prostate and has not spread to other parts of the body. Localised prostate cancer is also known as early or organ-confined prostate cancer. In a particular embodiment, the prostate cancer is advanced prostate cancer. Advanced prostate cancer refers to stage IV (T4N0M0, N1M0 or M1) hormone-sensitive prostate cancer and recurrent prostate cancer after treatment with curative intent, as well as castration-resistant prostate cancer (Komura K et al. Current treatment strategies for advanced prostate cancer. Int J Urol. 2018 Mar; 25(3):220-231). In a particular embodiment, the prostate cancer is recurrent or metastasic prostate cancer refers to prostate cancer that has spread beyond the prostate and pelvic lymph nodes (www.ncbi.nlm.nih.gov/books/NBK248412/).

In a preferred embodiment, the patient is a patient in which a primary prostate tumour has been surgically excised. In another preferred embodiment, the patient has not been treated with adjuvant therapy, such as chemotherapy or radiotherapy prior to the determination of the expression levels of the gene or genes of interest.

In prostate cancer therapy, a variety of treatments can be used in an attempt to eliminate or contain the cancer. Treatment for prostate cancer may involve Active Surveillance (AS), hormonal therapy, radiation therapy including brachytherapy (prostate brachytherapy) and external beam radiation, High Intensity Focused Ultrasound (HIFU), chemotherapy, or some combination. Which option is best depends on the stage of the disease, the Gleason score, and the PSA level. Other important factors are the man's age, his general health, and his feelings about potential treatments and their possible side effects. Because all treatments can have significant side effects, such as erectile dysfunction and urinary incontinence, treatment discussions often focus on balancing the goals of therapy with the risks of lifestyle alterations.

Briefly, depending on the above mentioned factors (e.g., age of the subject, as well as the size, location and cancer phase), (i) cytotoxic/cytostatic treatments, such as chemotherapy, which uses medicinal products against cancer to destroy the cancerous cells upon making the medicinal products circulate through the body through the blood vessels; radiotherapy, which uses high energy radiations to kill the cancer cells, and anti-tumour agents; and/or (ii) immunotherapy, wherein the administered compound stimulates, enhances or strengthens the natural function of the immune system against cancer to recognize and eliminate the cancerous cells from the body, can be used. Thus, the method of the invention allows determining the response of the subject having prostate cancer to any treatment, particularly, to any cytotoxic and/or cytostatic treatment and, more specifically, to a treatment by means of chemotherapy, radiotherapy, anti-tumour agents or combinations thereof.

Suitable chemotherapy agents include but are not limited to alkylating agents [e.g., Cisplatin, Carboplatin, Oxaliplatin, BBR3464, Chlorambucil, Chlormethine, Cyclophosphamides, Ifosmade, Melphalan, Carmustine, Fotemustine, Lomustine, Streptozocin, Busulfan, Dacarbazine, Mechlorethamine, Procarbazine, Temozolomide, ThioTPA, Uramustine, etc.]; anti-metabolites [e.g., purine (azathioprine, mercaptopurine), pyrimidine (Capecitabine, Cytarabine, Fluorouracil, Gemcitabine),folic acid (Methotrexate, Pemetrexed, Raltitrexed), etc.]; vinca alkaloids [e.g., Vincristine, Vinblastine, Vinorelbine, Vindesine, etc.]; a taxane [e.g., paclitaxel, docetaxel, BMS-247550, etc.]; an anthracycline [e.g., Daunorubicin, Doxorubicin, Epirubicin, Idarubicin, Mitoxantrone, Valrubicin, Bleomycin, Hydroxyurea, Mitomycin, etc.]; a topoisomerase inhibitor [e.g., Topotecan, Irinotecan Etoposide, Teniposide, etc.]; a monoclonal antibody [e.g., Alemtuzumab, Bevacizumab, Cetuximab, Gemtuzumab, Panitumumab, Rituximab, Trastuzumab, etc.]); a photosensitizer [e.g., Aminolevulinic acid, Methyl aminolevulinate, Porfimer sodium, Verteporfin, etc.]; a tyrosine kinase inhibitor [e.g., Gleevec(TM)]; an epidermal growth factor receptor inhibitor [e.g., Iressa(TM), erlotinib (Tarceva(TM)), gefitinib, etc.]; an FPTase inhibitor [e.g., FTIs (RI 15777, SCH66336, L-778, 123), etc.]; a KDR inhibitor [e.g., SU6668, PTK787, etc.]; a proteosome inhibitor [e.g., PS341, etc.]; a TS/DNA synthesis inhibitor [e.g., ZD9331, Raltirexed (ZD 1694, Tomudex), ZD9331, 5-FU, etc.]; an S-adenosyl-methionine decarboxylase inhibitor [e.g., SAM468A, etc.]; a DNA methylating agent [e.g., TMZ, etc.]; a DNA binding agent [e.g., PZA, etc.]; an agent which binds and inactivates 06-alkylguanine AGT [e.g., BG]; a c-ra/- I antisense oligo-deoxynucleotide [e.g., ISIS-5132 (CGP- 69846A)]; tumour immunotherapy; a steroidal and/or nonsteroidal antiinflammatory agent [e.g., corticosteroids, COX-2 inhibitors]; or other agents such as Alitretinoin, Altretamine, Amsacrine, Anagrelide, Arsenic trioxide, Asparaginase, Bexarotene, Bortezomib, Celecoxib, Dasatinib, Denileukin Diftitox, Estramustine, Hydroxycarbamide, Imatinib, Pentostatin, Masoprocol, Mitotane, Pegaspargase, and Tretinoin. Suitable chemotherapy agents are described with more detail in the literature, such as in The Merck Index in CD-ROM, 13th edition.

The term "adjuvant chemotherapy" as used herein means treatment of cancer with standard chemotherapeutic agents after surgery where all detectable disease has been removed, but where there still remains a risk of small amounts of remaining cancer. Adjuvant therapy can include chemotherapy or radiotherapy.

The term "chemotherapy" refers to the use of drugs to eliminate cancer cells. The drugs are generally administered through oral or intravenous route. Sometimes, chemotherapy is used together with radiation treatment.

The term "radiotherapy" or "radiotherapeutic treatment" is a term commonly used in the art to refer to multiple types of radiation therapy including internal and external radiation therapies or radio immunotherapy, and the use of various types of radiations including X-rays, gamma rays, alpha particles, beta particles, photons, electrons, neutrons, radioisotopes, and other forms of ionizing radiations.

The term "treatment", as used herein, refers to any type of therapy, which is aimed at terminating, preventing, ameliorating or reducing the susceptibility to a clinical condition as described herein. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, or immune deficiency.

When the cancer has metastasized, systemic treatments including but not limited to chemotherapy, hormone treatment, immunotherapy, or a combination thereof are used. Additionally, radiotherapy and/or surgery can be used. The choice of treatment generally depends on the type of primary cancer, the size, the location of the metastasis, the age, the general health of the patient and the types of treatments used previously.

All the terms and embodiments described elsewhere herein are equally applicable to this aspect of the invention.

A method of treatment of advanced, recurrent or metastatic prostate cancer to a patient in need thereof

In a fourth aspect, the invention relates to a method of treatment of low risk, indolent, localised, advanced, recurrent or metastatic prostate cancer to a patient in need thereof which comprises the administration of a therapy to said patient, wherein the patient is selected by a method which comprises determining, in a sample from the patient, the ratio between the average expression levels of a first gene signature and the average expression levels of a second gene signature,

(a) wherein the first gene signature is selected from the group consisting of HI1 and HI2, wherein signature HI1 consists of genes CPOX, GAL3St4, ORAI2, NIPA1; and wherein signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein an increased ratio between the average expression levels of the first gene signature and the average expression levels of the second gene signature with respect to a reference value is indicative that the patient is selected for treatment for prostate cancer.

Appropriate method steps to select a patient in need of treatment of advanced, recurrent or metastatic prostate cancer have been described elsewhere herein. Suitable methods of treatment of advanced, recurrent or metastatic prostate cancer have also been described elsewhere herein.

All the terms and embodiments described elsewhere herein are equally applicable to these aspects of the invention.

Kits and assay devices

In a fifth aspect, the invention relates to a kit or assay device comprising reagents adequate for the determination of the average expression level of a gene signature selected from the group consisting of HI1, HI2, LO1, LO2 and LO3, wherein the reagents are selected from the group of a set probes which specifically hybridize to the mRNA of said genes and a set of primer pairs which are capable of specifically amplifying the mRNAs of said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

In a sixth aspect, the invention also relates to a kit or assay device comprising reagents adequate for the determination of the average expression level of the polypeptides encoded by the genes in a gene signature selected from the group consisting of HI1, HI2, LO1, LO2 and LO3, wherein the reagents are a set of antibodies which specifically bind to the polypeptides encoded by said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

In the context of the present invention, "kit" or "assay device" is understood as a product or device containing the different reagents necessary for carrying out the methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers.

The reagents for use in the first or second methods of the invention may be formulated as a "kit" and thus, may be combined with one or more other types of elements or components (e.g., other types of biochemical reagents, containers, packages such as packaging intended for commercial sale, substrates to which the reagents are attached, electronic hardware components, etc.).

In a preferred embodiment, the reagents adequate for the determination of the expression levels of one or more genes comprise at least 10%, at least 20%, at least 30%, at least 40%, at least 50%, at least 60%, at least 70%, at least 80%, at least 90% or at least 100% of the total amount of reagents adequate for the determination of the expression levels of genes forming the kit. Thus, in the particular case of kits comprising reagents for the determination of the average expression levels of HI1 (i.e., the CPOX, GAL3St4, ORAI2 and NIPA1 genes), HI2 (i.e., the KHK, NOX4, PTGES3 and RRM2 genes), LO1 (i.e., the ATP8B1, CDO1, CHRNA2, GLB1L3, GNE, KATNAL2, PTGIS, SLC6A14, TP53INP2, and TRIM47 genes), LO2 (i.e., the ASPA, CDO1, CYP3A5, GLB1L2, GNE, ITPKC, KCTD14, PAH, PHYHD1, SIRT1, SLC40A1, SLC7A4, and SRD5A genes), LO3 (i.e., the ASPA, CDO1, GFPT2, GNE, ITPKC, PAH, SIRT1 and TPMT genes), the reagents specific for said genes (i.e. probes which are capable of hybridizing under stringent conditions to HI1 (i.e., the CPOX, GAL3St4, ORAI2 and NIPA1 genes), HI2 (i.e., the KHK, NOX4, PTGES3 and RRM2 genes), LO1 (i.e., the ATP8B1, CDO1, CHRNA2, GLB1L3, GNE, KATNAL2, PTGIS, SLC6A14, TP53INP2, and TRIM47 genes), LO2 (i.e., the ASPA, CDO1, CYP3A5, GLB1L2, GNE, ITPKC, KCTD14, PAH, PHYHD1, SIRT1, SLC40A1, SLC7A4, and SRD5A genes), LO3 (i.e., the ASPA, CDO1, GFPT2, GNE, ITPKC, PAH, SIRT1 and TPMT genes)) comprise at least 10%, at least 20%, at least 30%, at least 40%, at least 50%, at least 60%, at least 70%, at least 80%, at least 90% or at least 100% of the probes present in the kit. In further embodiments, the reagents adequate for the determination of the expression levels of one or more genes comprise at least 55% at least 60%, at least 65%, at least 70%, at least 75%, at least 80%, at least 90%, at least 95%, at least 96%, at least 97%, at least 98% or at least 99% of the total amount of reagents forming the kit.

In a particular example, the kit or assay device comprises reagents adequate for the determination of the average expression level of the HI1 gene signature. In a particular example, the kit or assay device comprises reagents adequate for the determination of the average expression level of the HI2 gene signature. In a particular example, the kit or assay device comprises reagents adequate for the determination of the average expression level of the LO1 gene signature. In a particular example, the kit or assay device comprises reagents adequate for the determination of the average expression level of the LO2 gene signature. In a particular example, the kit or assay device comprises reagents adequate for the determination of the average expression level of the LO3 gene signature.

In a further aspect, the invention relates to a kit or assay device comprises reagents adequate for the determination of the average expression level of a first gene signature and a second gene signature wherein the first gene signature is selected from the group consisting of HI1 and HI2, wherein gene signature HI1 consists of genes CPOX, GAL3St4, ORAI2 and NIPA1; and wherein gene signature HI2 consists of genes KHK, NOX4, PTGES3 and RRM2; wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47, wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

In a particular embodiment, the kit or assay device comprises reagent adequate for the determination of the average expression level of a first gene signature and a second gene signature, wherein the first gene signature is HI1 (CPOX, GAL3St4, ORAI2 and NIPA1) and wherein the second gene signature is LO1 (ATP8B1, CDO1, CHRNA2, GLB1L3, GNE, KATNAL2, PTGIS, SLC6A14, TP53INP2, and TRIM47).

In a particular embodiment, the kit or assay device comprises reagent adequate for the determination of the average expression level of a first gene signature and a second gene signature, wherein the first gene signature is HI1 (CPOX, GAL3St4, ORAI2 and NIPA1) and wherein the second gene signature is LO2 (ASPA, CDO1, CYP3A5, GLB1L2, GNE, ITPKC, KCTD14, PAH, PHYHD1, SIRT1, SLC40A1, SLC7A4, and SRD5A).

In a particular embodiment, the kit or assay device comprises reagent adequate for the determination of the average expression level of a first gene signature and a second gene signature, wherein the first gene signature is HI1 (CPOX, GAL3St4, ORAI2 and NIPA1) and wherein the second gene signature is LO3 (ASPA, CDO1, GFPT2, GNE, ITPKC, PAH, SIRT1 and TPMT).

In a particular embodiment, the kit or assay device comprises reagent adequate for the determination of the average expression level of a first gene signature and a second gene signature, wherein the first gene signature is HI2 (KHK, NOX4, PTGES3 and RRM2) and wherein the second gene signature is LO1 (ATP8B1, CDO1, CHRNA2, GLB1L3, GNE, KATNAL2, PTGIS, SLC6A14, TP53INP2, and TRIM47).

In a particular embodiment, the kit or assay device comprises reagent adequate for the determination of the average expression level of a first gene signature and a second gene signature, wherein the first gene signature is HI2 (KHK, NOX4, PTGES3 and RRM2) and wherein the second gene signature is LO2 (ASPA, CDO1, CYP3A5, GLB1L2, GNE, ITPKC, KCTD14, PAH, PHYHD1, SIRT1, SLC40A1, SLC7A4, and SRD5A).

In a particular embodiment, the kit or assay device comprises reagent adequate for the determination of the average expression level of a first gene signature and a second gene signature, wherein the first gene signature is HI2 (KHK, NOX4, PTGES3 and RRM2) and wherein the second gene signature is LO3 (ASPA, CDO1, GFPT2, GNE, ITPKC, PAH, SIRT1 and TPMT).

In a particular embodiment of the kit of the invention, the reagents of the kit are nucleic acids which are capable of specifically detecting the mRNA level of the genes mentioned above and/or the level of proteins encoded by one or more of the genes mentioned above. Nucleic acids capable of specifically hybridizing with the genes mentioned above can be one or more pairs of primer oligonucleotides for the specific amplification of fragments of the mRNAs (or of their corresponding cDNAs) of said genes. In a preferred embodiment, the first component of the kit of the invention comprises probes which can specifically hybridize to the genes mentioned above. The term "specifically hybridizing", as used herein, refers to conditions which allow hybridizing of two polynucleotides under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and the hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 μg/ml), 0.1% SDS, and 10% dextran sulfate at 42°C., with washes at 42°C in 0.2xSSC (sodium chloride/sodium citrate) and 50% formamide, followed by a high-stringency wash consisting of 0.1xSSC containing EDTA at 55 °C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and % SDS) less stringent that those described above. An example of moderately stringent conditions is overnight incubation at 37°C. in a solution comprising: 20% formamide, 5xSSC (150 mMNaCl, 15 mMtrisodium citrate), 50 mM sodium phosphate (pH 7.6), 5x Denhardt's solution, 10% dextran sulfate, and 20 mg/ml denatured sheared salmon sperm DNA, followed by washing the filters in 1xSSC at about 37-50°C. The skilled artisan will recognize how to adjust the temperature, ionic strength, etc. as necessary to accommodate factors such as probe length and the like.

In the event that the expression levels of several of the genes identified in the present invention are to be simultaneously determined, it is useful to include probes for all the genes the expression of which is to be determined in a microarray hybridization.

The microarrays comprise a plurality of nucleic acids that are spatially distributed and stably associated to a support (for example, a biochip). The nucleic acids have a sequence complementary to particular sub-sequences of genes the expression of which is to be detected, therefore are capable of hybridizing with said nucleic acids. In the methods of the invention, a microarray comprising an array of nucleic acids is put into contact with a preparation of nucleic acids isolated from the patient object of the study. The incubation of the microarray with the preparation of nucleic acids is carried out in conditions suitable for the hybridization. Subsequently, after the elimination of the nucleic acids which have not been retained in the support, the hybridization pattern is detected, which provides information on the genetic profile of the sample analyzed. Although the microarrays are capable of providing both qualitative and quantitative information of the nucleic acids present in a sample, the invention requires the use of arrays and methodologies capable of providing quantitative information.

The invention contemplates a variety of arrays with regard to the type of probes and with regard to the type of support used. The probes included in the arrays that are capable of hybridizing with the nucleic acids can be nucleic acids or analogues thereof which maintain the hybridization capacity such as for example, nucleic acids in which the phosphodiester bond has been substituted with a phosphorothioate, methylimine, methylphosphonate, phosphoramidate, guanidine bond and the like, nucleic acids in which the ribose of the nucleotides is substituted with another hexose, peptide nucleic acids (PNA). The length of the probes can of 5 to 50 nucleotides and, preferably, of 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or doublestranded nucleic acids. The array can contain all the specific probes of a certain mRNA of a certain length or can contain probes selected from different regions of an mRNA. Each probe is assayed in parallel with a probe with a changed base, preferably in a central position of the probe. The array is put into contact with a sample containing nucleic acids with sequences complementary to the probes of the array and the signal of hybridization with each of the probes and with the corresponding hybridization controls is determined. Those probes in which a higher difference is observed between the signal of hybridization with the probe and its hybridization control are selected. The optimization process can include a second round of optimization in which the hybridization array is hybridized with a sample that does not contain sequences complementary to the probes of the array. After the second round of selection, those probes having signals of hybridization lower than a threshold level will be selected. Thus, probes which pass both controls, i.e., which show a minimum level of unspecific hybridization and a maximum level of specific hybridization with the target nucleic acid are selected.

The microarrays of the invention contain not only specific probes for the polynucleotides indicating a determined pathophysiological situation, but also containing a series of control probes, which can be of three types: normalization controls, expression level controls and hybridization controls.

Probes suitable for use as expression controls correspond to genes expressed constitutively, such as genes encoding proteins which exert essential cell functions such as β-2-microglobulin, ubiquitin, ribosomal protein 18S, cyclophilin A, transferrin receptor, actin, GAPDH, tyrosine 3-monooxygenase/tryptophan 5-monooxygenase activation protein (YWHAZ), HPRT, and IPO8.

Once a set of probes showing the suitable specificity and a set of control probes are provided, the latter are arranged in the array in a known position such that, after the steps of hybridization and of detection, it is possible to establish a correlation between a positive signal of hybridization and the particular gene from the coordinates of the array in which the positive signal of hybridization is detected.

The microarrays can be high density arrays with thousands of oligonucleotides by means of photolithographic in situ synthesis methods (Fodor et al., 1991, Science, 767-773). This type of probe is usually redundant, i.e., they include several probes for each mRNA which is to be detected. In a preferred embodiment, the arrays are low density arrays or LDA containing less than 10000 probes per square centimetre. In said low density arrays, the different probes are manually applied with the aid of a pipette in different locations of a solid support (for example, a crystal surface, a membrane). The supports used to fix the probes can be obtained from a large variety of materials, including plastic, ceramics, metals, gels, membranes, crystals and the like. The microarrays can be obtained using any methodology known for the person skilled in the art.

In the event that the expression levels of the genes according to the present invention is determined by measuring the levels of the polypeptide or polypeptides encoded by said gene or genes, the kits according to the present invention comprise reagents which are capable of specifically binding to said polypeptide or polypeptides. For this purpose, the arrays of antibodies such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful. The antibodies of the array include any immunological agent capable of binding to a ligand with high affinity, including IgG, IgM, IgA, IgD and IgE, as well as molecules similar to antibodies which have an antigen binding site, such as Fab', Fab, F(ab')2, single domain antibodies or DABS, Fv, scFv and the like. The techniques for preparing said antibodies are very well known for the person skilled in the art and include the methods described by Ausubel et al. (Current Protocols in Molecular Biology, eds. Ausubel et al, John Wiley & Sons (1992)).

The antibodies of the array can be applied at high speed, for example, using commercially available robotic systems (for example, those produced by Genetic Microsystems or Biorobotics). The substrate of the array can be nitrocellulose, plastic, crystal or can be of a porous material as for example, acrylamide, agarose or another polymer. In another embodiment, it is possible to use cells producing the specific antibodies for detecting the proteins of the invention by means of their culture in array filters. After the induction of the expression of the antibodies, the latter are immobilized in the filter in the position of the array where the producing cell was located. An array of antibodies can be put into contact with a labelled target and the binding level of the target to the immobilized antibodies can be determined. If the target is not labelled, a sandwich type assay can be used in which a second labelled antibody specific for the polypeptide which binds to the polypeptide which is immobilized in the support is used. The quantification of the amount of polypeptide present in the sample in each point of the array can be stored in a database as an expression profile. The array of antibodies can be produced in duplicate and can be used to compare the binding profiles of two different samples.

The invention also relates to the use of a kit or assay device as described above for predicting the outcome of a patient suffering from prostate cancer or for predicting the response to therapy of a patient suffering from prostate cancer.

All the terms and embodiments previously described are equally applicable to these aspects of the invention.

## EXAMPLES

The following invention is hereby described by way of the following examples, which are to be construed as merely illustrative and not limitative of the scope of the invention.

## Example 1: Identification of a gene signature with prognostic value

The inventors have determined an improved gene signature with prognostic capacity using the Genetic Algorithm (GA) tool. Some basic concepts related to this technique are described herein. Genetic Algorithm (GA) is a search-based optimization technique based on the principles of Genetics and Natural Selection. It is frequently used to solve optimization problems, in research, and in machine learning. In GAs, we have a pool or a population of possible solutions to the given problem. These solutions then undergo recombination and mutation (like in natural genetics), producing new children (*population*), and the process is repeated over various generations (*iterations*). Each individual (or candidate solution) is assigned a fitness value (based on its objective function value) and the fitter individuals are given a higher chance to mate and yield more "fitter" individuals. In this way better individuals or solutions keep "evolving" over generations, till a stopping criterion is reached.

This technique was used to find among some sets of genes (or *"universes"*) some subsets of genes (or *"signatures"*), which differentiate between patients with prostate cancer that recur and those who do not recur.

These universes have been obtained by different methodologies:

1. An array was made in which human samples with PGC1A gene downregulated were compared against normal ones. The correlation between the PGC1A gene and those differentially expressed genes was calculated. Genes that directly correlate with PGC1A were defined as a universe.

2. Metabolic genes were studied to verify their overexpression or subexpression were related to the risk of recurrence. From almost 2,800 metabolic genes, it was found that the overexpression of 98 of them is linked to recurrence, while the underexpression of another 93 is also linked to recurrence. Thus, these two sets of genes were defined as two independent universes.

3. GA was launched independently using universes from points 1 and 2. For both of them, genes that appear in the three signatures with better values of Hazard Ratio (HR) in all the loops of genetic algorithm that have been carried out, were chosen to make a new universe.

4. As an improvement of the previous approach, the genes that appear on the top 3 signatures obtained for universe 1 were joined with the 93 metabolic genes whose underexpression is linked to recurrence. Apart from this, the 98 genes whose overexpression is linked to recurrence was establish as a universe.

Regarding the values that we seek to improve in each iteration of the genetic algorithm, we will use the Hazard Ratio (HR) which is the ratio of the hazard rates corresponding to the conditions described by two levels of an explanatory variable and the Negative Predictive Value (NPV), which is the probability that subjects with a negative screening test truly do not have the disease. Both values will be used independently in the genetic algorithm to get the optimal signatures, but analyzed in common in order to choose the final ones. Universes obtained from approaches 1 and 2 were used to optimize the HR, while universes from 3 and 4, were used to optimize the NPV.

For the calculation of these values, the patient population will be divided according to the average gene expression levels into quartiles, comparing those patients that are in the Q1 (the lowest) against the rest of the samples, or the Q4 (the highest) against the rest of the samples. To check which set of genes has the higher HR, patients with primary tumours from two large and well-annotated prostate cancer databases (Taylor et al, Cancer Cell, 2010; and TCGA (The Cancer Genome Atlas)) have been used. In order to determine the HR, the ratio between the average signal value of each gene signature combination has been measured per patient and established three different levels: Q1 (quartile 1) represents 25% of patients exhibiting the lowest average signal value of the gene signature; rest, quartiles 2 to 4; and Q4 (quartile 4) represents 25% of patients exhibiting the highest average signal value of the gene signature. Regarding the fitness function, the HR has been defined as the minimum valid value of each chromosome, being discarded those chromosomes with smaller values.

Methods

After some tests, the inventors established the population size (the number of different solutions in each generation) in 100 and determined 1000 iterations for every simulation (1000 different generations). Also, as each execution of the algorithm returned a set of signatures based on a single common root (called "initial population"), a minimum of 25 executions (repetitions) of the script was established in order to obtain signatures from different origin. The initialization of these executions can be in two ways:

- **Random Initialization:** The initial population comes from completely random solutions.

- **Heuristic initialization:** The initial population comes a predefined set of genes.

To check which set of genes has the higher HR or NPV, the average signal value of each signature was measured per patient, and used to separate them by quartiles. Regarding the fitness function, in the case of heuristic initialization, the inventors defined the HR and NPV of the initial population as the minimum valid value. In case a solution obtained a smaller value, it was discarded. In the case of random initialization, there is no HR or NPV defined as the minimum and the fitness function only tries to improve the obtained values.

As it was mentioned before, due to the randomness of this kind of algorithms, the inventors launched each simulation 25 times, and the 10 best options per each simulation were chosen. As an output of every one we have obtained the 10 subsets of genes with higher HR or NPV, as well as other statistical results. Finally, the inventors selected the 100 signatures with higher HR or NPV in each dataset (Taylor or TCGA), and proved them on the other one in order to obtain two values per possible signature. The chosen signature was the one with higher values in both datasets.

The resulting signature is deconvoluted (which means that gene per gene, the inventors checked whether its deletion from the signature does or does not affect the results) as many times as needed, and used as a seed for the following GA. The full process is repeated as many times as the HR or NPV is improved.

Once a set of signatures have been chosen, a complete set of analyses has been performed by the team. Studying the results, five signatures have been selected from the full set. Of these five signatures, two of the signatures correspond to genes that are upregulated, while three of the signatures correspond to genes that are downregulated. The gene signatures that are upregulated are HI1 (the CPOX, GAL3St4, ORAI2 and NIPA1 genes) and HI2 (the KHK, NOX4, PTGES3 and RRM2 genes). The genes signatures that are downregulated are LO1 (the ATP8B1, CDO1, CHRNA2, GLB1L3, GNE, KATNAL2, PTGIS, SLC6A14, TP53INP2, and TRIM47 genes); LO2 (the ASPA, CDO1, CYP3A5, GLB1L2, GNE, ITPKC, KCTD14, PAH, PHYHD1, SIRT1, SLC40A1, SLC7A4, and SRD5A genes); and LO3 (the ASPA, CDO1, GFPT2, GNE, ITPKC, PAH, SIRT1 and TPMT genes).

Finally, the gene signatures are combined to obtain the ratio between the average expression levels of a first gene signature (upregulated) and the average expression levels of a second gene signature (downregulated). These gene signature combinations yield the most informative results. The six gene signature combinations of the invention are:

- HI1_LO1;
- HI1_LO2;
- HI1_LO3;
- H12_LO1;
- HI2_LO2;
- H12_LO3.

**Results**

The prognostic potential of the gene signature combinations has been established against the full Taylor and TCGA databases and the results of the calculations are shown in Tables 1-4 below. In particular, the Smooth Hazard Ratio (SHR) is calculated for patients with all Prostate Specific Antigen (PSA) levels, and results are represented for prostate tumour (PT) patients (Fig. 1A) and for Gleason Score 6 (GS6) and Gleason Score 7 (GS7) patients (Fig. 1B).

Further, the Negative predictive value (NPV) is calculated in order to represent ROC curves for patients with all Prostate Specific Antigen (PSA) levels, where results are separately presented for prostate tumour (PT) patients (Fig. 2A) and for Gleason Score 6 (GS6) and Gleason Score 7 (GS7) patients (Fig. 2B). Negative predictive value (NPV) is the probability that subjects with a negative screening test truly do not have the disease. The values for the signatures of the invention are from 0.97-0.99 while competitors have a value of 0.90.

$$NPV = \frac{\text{number of true negatives}}{\text{number of true negatives} + \text{number of false negatives}} = \frac{\text{number of true negatives}}{\text{number of negative calls}}$$

In addition, Kaplan Meier analyses for all PSA patients (Fig. 3A) or for Gleason Score GS6 and GS7 patients (Fig. 3B) are performed. This type of analysis shows the differences into the relapse of the disease among different subgroups of the population. These subgroups are obtained by separating and comparing the patients in the four different quartiles (Q1 to Q4). This type of analysis is used to estimate survival and presents survival data. It shows the differences in the relapse of the disease among different subgroups of the population with time. These subgroups are obtained by separating and comparing the patients in the four different quartiles according to the gene expression ratio being quartile 1 (Q1) the lowest gene expression ratio, and quartile 4 (Q4) the highest ratio. Graphics show the stratification of the different populations made by the inventors' gene signature and gene signature combinations. In these results, it can be seen that the rate of survival decreases with higher gene expression ratios.

For comparison with diagnosis by simply determining PSA levels, heat maps are prepared for all PSA patients in the Taylor dataset (Fig. 4A) and in the TCGA dataset (Fig. 4B). The heat maps represent the ratio between the average expression levels of two specific gene signatures, as indicated. Higher ratios represent a higher risk of recurrence (on the top of the graphic). Red lines are recurrence patients with low PSA. Green lines are recurrence patients with high PSA. As it can be seen, there is not necessarily a correlation between PSA levels and the ratio between the average expression levels of the two specific gene signatures. This can particularly be seen in the case of low PSA patients when they are stratified according to the ratio between signatures HI2 and LO2 (Fig. 5A) and to the ratio between signatures HI2 and LO3 (Fig. 5B). Red bars represent recurrent patients with PSA<10.

Finally, the six gene signature combinations may all be simultaneously performed whereby more informative data about a patient is collected. Fig. 6A shows true positive and false negative results for all signature ratios in the Taylor dataset. Of 27 patients who show recurrence in prostate tumour (PT), 26 are detected by at least one signature ratio, while 19 are detected in all signature ratios. On the other hand, Fig. 6B shows true negative and false positive results for all signature ratios in the Taylor dataset. Of 104 patients who show no recurrence in prostate tumour (PT), 80 are correctly assigned by at least one signature ratio, while 38 are correctly assigned in all signature ratios.

**Example 2: Clinical applications of the gene signature ratio**

The inventors have named their five gene signatures and six gene signature combinations PROMETAMARK. PROMETAMARK is a dual function biomarker based on gene expression of metabolic genes and regulators. The inventors have applied machine learning and genetic algorithms to aid the discovery and implementation of gene expression-based prostate cancer biomarkers. This analysis has resulted in a gene expression-based biomarker (PROMETAMARK) with two distinctive clinically-relevant applications:

1. Identification of indolent prostate cancers. We have applied genetic algorithms that stratify patients with low-risk prostate cancer. Since empirical data and biological material for molecular studies derived from patients subjected to AS is still scarce, we have applied as outputs the success of surgery in prostate cancers with low or intermediate grade disease (Gleason 6 and 7, ISUP G1-G3). Our machine learning strategy has led us to define metabolic signatures that are highly potent in discriminating patients with good prognosis. When incorporating PSA at diagnosis as an additional parameter, our biomarker (INDOMETAB) reaches negative predictive values (NPV) greater than 97% in two independent patient cohorts, which exceeds any commercial biomarker designed for this purpose. Based on this data, we propose that the biomarker has the potential to serve as a device to molecularly classify prostate cancer patients based on core biopsies in protocols of AS.

2. Identification of aggressive prostate cancers. The use of genetic algorithms focused on obtaining a higher differential risk of biochemical recurrence in prostate cancer has resulted in a genetic signature that shows a progressive increase in risk with the elevation of AGGREMETAB score. Patients with the highest AGGREMETAB scores increase the risk of recurrence after surgery between 15 and 20 fold compared to those with low AGGREMETAB score. The ROC value of this signature is 0.81.

Table 1: ALL PSA, TAYLOR DATASET

|  |  | Taylor | | | | |
|  |  | SHR | | by ROC | Q4 vs Rest | |
|  | Name | Q4 vs Rest | Q1 vs Q4 | NPV | Sensitivity | Specificity |
|---|---|---|---|---|---|---|
| PT | HI1_LO2 | 6,833916 | 22,84098696 | 0,925 | 0,592593 | 0,836538 |
|  | HI1_LO3 | 6,152509 | 20,87111527 | 0,921348 | 0,592593 | 0,836538 |
|  | HI1_LO1 | 7,290711 | 907311248,1 | 0,921053 | 0,592593 | 0,836538 |
|  | HI2_LO2 | 6,164963 | 21,31719524 | 0,952941 | 0,592593 | 0,836538 |
|  | HI2_LO3 | 8,14474 | 23,65647483 | 0,953488 | 0,666667 | 0,855769 |
|  | HI2_LO1 | 5,815688 | 18,6746023 | 0,946667 | 0,592593 | 0,836538 |
| GS6 and GS7 | HI1_LO2 | 2,178756 | 6,195432332 | 0,940299 | 0,4375 | 0,777778 |
|  | HI1_LO3 | 4,021694 | 9,131467231 | 0,945205 | 0,5625 | 0,79798 |
|  | HI1_LO1 | 2,57909 | 590856168,4 | 0,953846 | 0,4375 | 0,777778 |
|  | HI2_LO2 | 3,885413 | 9,46022736 | 0,951807 | 0,5625 | 0,79798 |
|  | HI2_LO3 | 8,352483 | 12,92962049 | 0,963855 | 0,6875 | 0,818182 |
|  | HI2_LO1 | 3,238761 | 7,252067605 | 0,945205 | 0,5 | 0,787879 |

Table 2: ALL PSA, TCGA DATASET

|  |  | TCGA | | | | |
|  |  | SHR | | by ROC | Q4 vs Rest | |
|  | Name | Q4 vs Rest | Q1 vs Q4 | NPV | Sensitivity | Specificity |
|---|---|---|---|---|---|---|
| PT | HI1_LO2 | 5,634886 | 15,06608678 | 0,970149 | 0,508772 | 0,801444 |
|  | HI1_LO3 | 6,454513 | 811860231,8 | 0,954887 | 0,45614 | 0,794224 |
|  | HI1_LO1 | 7,598636 | 19,37971327 | 0,961832 | 0,45614 | 0,790614 |
|  | HI2_LO2 | 7,652757 | 8,500222436 | 0,958904 | 0,508772 | 0,801444 |
|  | HI2_LO3 | 4,409226 | 14,39138981 | 0,974576 | 0,473684 | 0,797834 |
|  | HI2_LO1 | 9,71589 | 18,07319244 | 0,985185 | 0,508772 | 0,801444 |
| GS6 and GS7 | HI1_LO2 | 9,215949 | 660497846,3 | 0,991228 | 0,384615 | 0,757895 |
|  | HI1_LO3 | 9,597013 | 612983644,3 | 0,991803 | 0,538462 | 0,768421 |
|  | HI1_LO1 | 9,39818 | 691402535,4 | 0,992126 | 0,384615 | 0,757895 |
|  | HI2_LO2 | 8,323087 | 3,061674852 | 0,990291 | 0,538462 | 0,768421 |
|  | HI2_LO3 | 8,323087 | 671030965,5 | 0,990741 | 0,538462 | 0,768421 |
|  | HI2_LO1 | 8,701363 | 663759415,6 | 0,99187 | 0,384615 | 0,757895 |

Table 3: LOW PSA (PSA<10), TAYLOR DATASET

| | | Taylor | | | | |
|---|---|---|---|---|---|---|
| | | SHR | | by ROC | Q4 vs Rest | |
| | Name | Q vs Rest | Q1 vs Q4 | NPV | Sensitivity | Specificity |
| | HI1_LO2 | 7,764239 | 1764495621 | 0,957143 | 0,684211 | 0,840909 |
| | HI1_LO3 | 7,697641 | 1902093186 | 0,957143 | 0,684211 | 0,840909 |
| | HI1_LO1 | 5,666403 | 701342482,9 | 0,95082 | 0,578947 | 0,818182 |
| | HI2_LO2 | 5,757275 | 1881722229 | 0,972973 | 0,631579 | 0,829545 |
| | HI2_LO3 | 10,54539 | 2026798898 | 0,961039 | 0,736842 | 0,852273 |
| PT | HI2_LO1 | 6,237032 | 639262558,3 | 0,953846 | 0,631579 | 0,829545 |
| | HI1_LO2 | 2,551986 | 457438460 | 0,956522 | 0,5 | 0,77907 |
| | HI1_LO3 | 4,112302 | 1432363282 | 0,955882 | 0,583333 | 0,790698 |
| | HI1_LO1 | 2,121552 | 578056573,1 | 0,964286 | 0,416667 | 0,767442 |
| | HI2_LO2 | 14,99718 | 1635038614 | 0,972603 | 0,833333 | 0,825581 |
| | HI2_LO3 | 17,57321 | 1958032292 | 0,972973 | 0,833333 | 0,825581 |
| GS6 and GS7 | HI2_LO1 | 4,76653 | 1493802537 | 0,953125 | 0,583333 | 0,790698 |

Table 4: LOW PSA (PSA<10), TCGA DATASET

| | | TCGA | | | | |
|---|---|---|---|---|---|---|
| | | SHR | | by ROC | Q4 vs Rest | |
| | Name | Q vs Rest | Q1 vs Q4 | NPV | Sensitivity | Specificity |
| | HI1_LO2 | 7,91551 | 8,092580873 | 0,970874 | 0,666667 | 0,777778 |
| | HI1_LO3 | 7,702373 | 769055979,7 | 0,972222 | 0,666667 | 0,777778 |
| | HI1_LO1 | 6,490627 | 7,068191073 | 0,9875 | 0,666667 | 0,777778 |
| | HI2_LO2 | 3,991571 | 5,427982641 | 0,975904 | 0,555556 | 0,769231 |
| | HI2_LO3 | 6,89992 | 7,149927515 | 0,978495 | 0,666667 | 0,777778 |
| PT | HI2_LO1 | 11,44543 | 7,291968399 | 0,98 | 0,777778 | 0,786325 |
| | HI1_LO2 | 6,374565 | 1687444110 | 0,988764 | 0,666667 | 0,762887 |
| | HI1_LO3 | 6,151142 | 679503776,1 | 0,989247 | 0,666667 | 0,762887 |
| | HI1_LO1 | 5,950824 | 646751361,7 | 1 | 0,666667 | 0,762887 |
| | HI2_LO2 | 5,950824 | 613303690,9 | 0,987179 | 0,666667 | 0,762887 |
| | HI2_LO3 | 5,950824 | 613303700,1 | 0,987952 | 0,666667 | 0,762887 |
| GS6 and GS7 | HI2_LO1 | 5,395963 | 542113117,5 | 0,989474 | 0,666667 | 0,762887 |

**Claims**

1. A method for predicting the outcome of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; and wherein gene signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of an increased risk of a negative outcome for the patient, or
(ii) a decreased ratio between the average expression levels of the first gene signature and the average expression levels of the second gene signature with respect to a reference value is indicative of a decreased risk of a negative outcome for the patient.

2. The method of claim 1, wherein the outcome of the patient is determined as time to cancer recurrence, overall survival, disease-specific survival, disease-free survival or occurrence of metastasis.

3. A method for predicting response to therapy of a patient suffering from prostate cancer, which comprises determining, in a sample from the patient, the ratio between the average expression levels of a first gene signature and the average expression levels of a second gene signature,

(a) wherein the first gene signature is selected from the group consisting of HI1 and HI2, wherein gene signature HI1 consists of genes CPOX, GAL3St4, ORAI2 and NIPA1; wherein gene signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein

(i) an increased ratio between the average expression levels of the first gene signature and the average expression levels of the second gene signature with respect to a reference value is indicative of a negative response of the patient to the therapy, or
(ii) a decreased ratio between the average expression levels of the first gene signature and the average expression levels of the second gene signature with respect to a reference value is indicative of a positive response of the patient to the therapy.

4. A method for selecting a patient to be treated for low risk, indolent, localised, advanced, recurrent or metastatic prostate cancer which comprises determining, in a sample from the patient, the ratio between the average expression levels of a first gene signature and the average expression levels of a second gene signature,

(a) wherein the first gene signature is selected from the group consisting of HI1 and HI2, wherein signature HI1 consists of genes CPOX, GAL3St4, ORAI2, NIPA1; and wherein signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT;

wherein an increased ratio between the average expression levels of the first gene signature and the average expression levels of the second gene signature with respect to a reference value is indicative that the patient is selected for treatment for prostate cancer.

**5.** The method of any one of claims 1 to 4 wherein the reference value is the mean ratio between the average expression levels of the first gene signature and the average expression levels of the second gene signature in a pool of samples from primary prostate tumours,

(a) wherein the first gene signature is selected from the group consisting of HI1 and HI2, wherein gene signature HI1 consists of genes CPOX, GAL3St4, ORAI2 and NIPA1; and wherein gene signature HI2 consists of genes KHK, NOX4, PTGES3 and RRM2;
(b) wherein the second gene signature is selected from the group consisting of LO1, LO2 and LO3, wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

**6.** The method of any one of claim 1 to 5, wherein the method further comprises the determination of Prostate Specific Antigen (PSA).

**7.** The method of any one of claim 1 to 6, wherein the sample is a sample containing tumour cells.

**8.** The method of claim 7, wherein the sample containing tumour cells is tumour tissue.

**9.** The method of claim 8, wherein the tumour tissue is a tumour biopsy or a surgically resected tumour.

**10.** The method of claim 9, wherein the patient is a patient in which a primary prostate tumour has been surgically excised.

**11.** The method of any one of claims 1 to 10, wherein the determination of the expression levels of the genes in the gene signatures is carried out by determining the levels of the corresponding mRNAs or by determining the levels of the polypeptides encoded by said genes.

**12.** A method of treatment of low risk, indolent, localised, advanced, recurrent or metastatic prostate cancer to a patient in need thereof which comprises the administration of a therapy to said patient, wherein the patient is selected by a method of any one of claims 4 to 11.

**13.** The method according to any one of claims 1-12, wherein all of gene signature ratios H1_LO1; H1_LO2; H1_LO3; H2_LO1; H2_LO2 and H2_LO3 are determined for the patient, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

**14.** A kit or assay device comprising reagents adequate for the determination of the average expression level of a gene signature selected from the group consisting of HI1, HI2, LO1, LO2 and LO3, wherein the reagents are selected from the group of a set probes which specifically hybridize to the mRNA of said genes and a set of primer pairs which are capable of specifically amplifying the mRNAs of said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMT.

**15.** A kit or assay device comprising reagents adequate for the determination of the average expression level of the polypeptides encoded by the genes in a gene signature selected from the group consisting of HI1, HI2, LO1, LO2 and LO3, wherein the reagents are a set of antibodies which specifically bind to the polypeptides encoded by said genes and wherein said reagents comprise at least 10% of the reagents present in the kit, wherein gene signature HI1 consists of genes CPOX, GAL3St4, ORAI2 and NIPA1; wherein gene signature HI2 consists of genes KHK, NOX4, PTGES3 and RRM2; wherein gene signature LO1 consists of ATP8B1, CDO1, CHRNA2, GLB1L3, GNE, KATNAL2, PTGIS, SLC6A14, TP53INP2, and TRIM47; wherein gene signature LO2 consists of ASPA, CDO1, CYP3A5, GLB1L2, GNE, ITPKC, KCTD14, PAH, PHYHD1, SIRT1, SLC40A1, SLC7A4, and SRD5A; and wherein gene signature LO3 consists of ASPA, CDO1, GFPT2, GNE, ITPKC, PAH, SIRT1 and TPMTN.

## Taylor: All PSA: PT

FIG. 1A

# TCGA: All PSA: PT

FIG. 1A (cont.)

FIG. 1B

# TCGA: All PSA: GS 6 + 7

FIG. 1B (cont.)

# Taylor: All PSA: PT

FIG. 2A

# TCGA: All PSA: PT

FIG. 2A (cont.)

# Taylor: All PSA: GS 6 + 7

**FIG. 2B**

## TCGA: All PSA: GS 6 + 7

### HI1 & LO1

### HI2 & LO1

### HI1 & LO2

### HI2 & LO2

### HI1 & LO3

### HI2 & LO3

**FIG. 2B (cont.)**

# Taylor: All PSA: PT

**FIG. 3A**

# TCGA: All PSA: PT

FIG 3A (cont.)

## Taylor: All PSA: GS 6 + 7

FIG. 3B

## TCGA: All PSA: GS 6 + 7

FIG. 3B (cont.)

# Taylor_All PSA

FIG. 4A

## TCGA_All PSA

FIG. 4B

Low PSA: black bars recurrent patients with PSA <10

FIG. 5A

EP 3 674 421 A1

**Low PSA**: black bars recurrent patients with PSA <10

(HI2_LO3 Ratio)

NPV=0.978
HR (Q1 vs Q4)= 7.149
HR (Q4 vs rest)= 6.899

FIG. 5B

Taylor, PT: true positive and false negative.

FIG. 6A

Taylor, PT: true negative and false positive.

FIG. 6B

Europäisches
Patentamt

European
Patent Office

Office européen
des brevets

# EUROPEAN SEARCH REPORT

Application Number

EP 18 38 3000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/021483 A2 (ORDWAY RES INST [US]; GLINKSY GENNADI V [US]) 21 February 2008 (2008-02-21) * page 50, paragraph 2 - page 51, paragraph 2 * * page 60, last paragraph - page 63, paragraph 2 * * table 4 * * figure 14 * * claims 1-24 * | 1-15 | INV. C12Q1/6886 |
| Y | E. K. MARKERT ET AL: "Molecular classification of prostate cancer using curated expression signatures", PNAS, vol. 108, no. 52, 28 November 2011 (2011-11-28), pages 21276-21281, XP055390712, US ISSN: 0027-8424, DOI: 10.1073/pnas.1117029108 * the whole document * | 1-15 | |
| Y | US 2004/053317 A1 (GLINSKII GUENNADI V [US]) 18 March 2004 (2004-03-18) * examples 1,11 * * paragraphs [0109] - [0132] * | 1-15 | TECHNICAL FIELDS SEARCHED (IPC) C12Q |
| Y | RUI LIU ET AL: "The Prognostic Role of a Gene Signature from Tumorigenic Breast-Cancer Cells", NEW ENGLAND JOURNAL OF MEDICINE, vol. 356, no. 3, 18 January 2007 (2007-01-18), pages 217-226, XP055005585, ISSN: 0028-4793, DOI: 10.1056/NEJMoa063994 * the whole document * | 1-15 | |

-/--

The present search report has been drawn up for all claims

| Place of search | Date of completion of the search | Examiner |
|---|---|---|
| The Hague | 21 June 2019 | Bruma, Anja |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 1 of 3

Europäisches
Patentamt

European
Patent Office

Office européen
des brevets

# EUROPEAN SEARCH REPORT

Application Number

EP 18 38 3000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2018/189292 A1 (INSTITUT NATIONAL DE LA SANTE ET DE LA RECH MEDICALE [FR] ET AL.) 18 October 2018 (2018-10-18) <br> * page 28 - page 30, paragraph 3 * <br> * claims 1-15 * | 1-15 | |
| Y | Ian Johnson ET AL: "Endosomal gene expression: a new indicator for prostate cancer patient prognosis?", Oncotarget, 10 November 2015 (2015-11-10), page 37919, XP055332941, United States DOI: 10.18632/oncotarget.6114 Retrieved from the Internet: URL:http://www.impactjournals.com/oncotarget/index.php?journal=oncotarget&page=article&op=download&path[]=6114&path[]=15079 * the whole document * | 1-15 | |
| Y | SEKAR VASANTHAKUMAR ET AL: "Molecular and Functional Diagnostic Tools in Precision Oncology for Urological Malignancies", INDIAN JOURNAL OF SURGICAL ONCOLOGY, SPRINGER INDIA, INDIA, vol. 8, no. 1, 15 December 2016 (2016-12-15), pages 24-32, XP036134164, ISSN: 0975-7651, DOI: 10.1007/S13193-016-0591-4 [retrieved on 2016-12-15] * the whole document * | 1-15 | TECHNICAL FIELDS SEARCHED (IPC) |

-/--

The present search report has been drawn up for all claims

| Place of search | Date of completion of the search | Examiner |
|---|---|---|
| The Hague | 21 June 2019 | Bruma, Anja |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**EUROPEAN SEARCH REPORT**

Europäisches
Patentamt
European
Patent Office
Office européen
des brevets

Application Number

EP 18 38 3000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | "Abstracts Accepted for Poster Presentations", ANNALS OF SURGICAL ONCOLOGY, SPRINGER-VERLAG, NE, vol. 16, no. 1, 13 January 2009 (2009-01-13), pages 31-123, XP019668080, ISSN: 1534-4681 * the whole document * | 1-15 | |
| Y | Mohammed Alshalafa ET AL: "Detecting Cancer Outlier Genes with Potential Rearrangement Using Gene Expression Data and Biological Networks", , 1 January 2012 (2012-01-01), XP055598434, Retrieved from the Internet: URL:https://www.hindawi.com/journals/abi/2012/373506/ [retrieved on 2019-06-21] * the whole document * | 1-15 | |

TECHNICAL FIELDS
SEARCHED      (IPC)

The present search report has been drawn up for all claims

| Place of search | Date of completion of the search | Examiner |
|---|---|---|
| The Hague | 21 June 2019 | Bruma, Anja |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P04C01)

page 3 of 3

## ANNEX TO THE EUROPEAN SEARCH REPORT
## ON EUROPEAN PATENT APPLICATION NO.

EP 18 38 3000

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2019

| Patent document cited in search report | | Publication date | Patent family member(s) | | Publication date |
|---|---|---|---|---|---|
| WO 2008021483 | A2 | 21-02-2008 | CA 2660857 A1 | | 21-02-2008 |
| | | | EP 2059615 A2 | | 20-05-2009 |
| | | | WO 2008021483 A2 | | 21-02-2008 |
| US 2004053317 | A1 | 18-03-2004 | AU 2003274970 A1 | | 30-04-2004 |
| | | | CA 2498418 A1 | | 25-03-2004 |
| | | | EP 1552293 A2 | | 13-07-2005 |
| | | | US 2004053317 A1 | | 18-03-2004 |
| | | | US 2005142573 A1 | | 30-06-2005 |
| | | | WO 2004025258 A2 | | 25-03-2004 |
| WO 2018189292 | A1 | 18-10-2018 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010030818 A
- WO 0140803 A
- WO 9951773 A1

### Non-patent literature cited in the description

- **PEROU et al.** *Nature,* 2000
- **VAN'T VEER et al.** *Nature,* 2002
- **KITTANEH et al.** *Biomark Cancer,* 2013
- **ZWEIG ; CAMPBELL.** Receiver-operating characteristic (ROC) plots: a fundamental evaluation tool in clinical medicine. *Clin Chem.,* April 1993, vol. 39 (4), 561-77
- database. ENSG00000108381
- database. ENSG00000081923
- database. ENSG00000129596
- database. ENSG00000120903
- database. ENSG00000080819
- database. ENSG00000106258
- database. ENSG00000197093
- database. ENSG00000131459
- database. ENSG00000149328
- database. ENSG00000166105
- database. ENSG00000159921
- database. ENSG00000086544
- database. ENSG00000167216
- database. ENSG00000151364
- database. ENSG00000138030
- database. ENSG00000086991
- database. ENSG00000160991
- database. ENSG00000171759
- database. ENSG00000175287
- database. ENSG00000110958
- database. ENSG00000124212
- database. ENSG00000171848
- database. ENSG00000096717
- database. ENSG00000138449
- database. ENSG00000268104
- database. ENSG00000099960
- database. ENSG00000277893
- database. ENSG00000078804
- database. ENSG00000137364
- database. ENSG00000132481
- **SAMBROOK, J. et al.** Molecular cloning: A Laboratory Manual. Cold Spring Harbor Laboratory Press, 2001, vol. 1-3
- **JASON A. REUTER et al.** High-Throughput Sequencing Technologies. *Mol Cell,* 21 May 2015, vol. 58 (4), 586-597
- database. ENSG00000142515
- **D'AMICO A.V. et al.** Biochemical outcome after radical prostatectomy, external beam radiation therapy, or interstitial radiation therapy for clinically localized prostate cancer. *JAMA,* 1998, vol. 280 (11), 969-74
- **BANGMA CH ; ROOBOL MJ.** Defining and predicting indolent and low risk prostate cancer. *Crit Rev Oncol Hematol.,* August 2012, vol. 83 (2), 235-41
- **KOMURA K et al.** Current treatment strategies for advanced prostate cancer. *Int J Urol.,* March 2018, vol. 25 (3), 220-231
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **FODOR et al.** *Science,* 1991, 767-773
- **DE WILDT et al.** *Nat. Biotechnol.,* 2000, vol. 18, 989-994
- **LUEKING et al.** *Anal. Biochem.,* 1999, vol. 270, 103-111
- **GE et al.** *Nucleic Acids Res.,* 2000, vol. 28 (e3), I-VII
- **MACBEATH ; SCHREIBER.** *Science,* 2000, vol. 289, 1760-1763
- **AUSUBEL et al.** Current Protocols in Molecular Biology. John Wiley & Sons, 1992
- **TAYLOR et al.** *Cancer Cell,* 2010